(19) **Europäisches Patentamt**
**European Patent Office**
**Office européen des brevets**

(11) **EP 3 040 715 B1**

(12) **EUROPEAN PATENT SPECIFICATION**

(45) Date of publication and mention
of the grant of the patent:
**05.02.2020 Bulletin 2020/06**

(51) Int Cl.:
***G01N 25/48*** *(2006.01)*     *G01N 33/28* *(2006.01)*

(21) Application number: **15397544.6**

(22) Date of filing: **15.12.2015**

(54) **A METHOD FOR MEASURING HEAT OR RATE OF HEAT RELEASE OF A SLOW REACTION OF A SUBSTANCE OR SUBSTANCES**

VERFAHREN ZUR MESSUNG VON WÄRME ODER DER RATE DER WÄRMEABGABE EINER LANGSAMEN REAKTION EINER SUBSTANZ ODER VON SUBSTANZEN

PROCÉDÉ DE MESURE DE LA CHALEUR OU DU TAUX DE LIBÉRATION DE CHALEUR D'UNE RÉACTION LENTE D'UNE SUBSTANCE OU DE SUBSTANCES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2014 FI 20146165**

(43) Date of publication of application:
**06.07.2016 Bulletin 2016/27**

(73) Proprietor: **Valmet Technologies Oy**
**02150 Espoo (FI)**

(72) Inventors:
• **KOKKO, Lauri**
**33710 TAMPERE (FI)**

• **RIIHIMÄKI, Teppo**
**37240 LINNAVUORI (FI)**
• **KAURA, Antti**
**33560 TAMPERE (FI)**
• **AUTIO, Joakim**
**36270 KANGASALA (FI)**

(74) Representative: **Berggren Oy, Tampere**
**Visiokatu 1**
**33720 Tampere (FI)**

(56) References cited:
**DE-A1- 4 414 082    US-A- 4 783 174**

**Description**

Field of the Invention

The invention relates to calorimetry of slow chemical reactions. The invention relates to a method for measuring heat produced by a slow reaction or the release rate of the heat of the reaction. The invention relates to a method for measuring heat produced by a slow reaction, which method is easily and cost effectively parallelizable. Moreover, the invention relates to a method for measuring heat produced by a spontaneous reaction of bio-oil at a reasonable low temperature regime, wherein the chemical reactions are slow. In particular, the invention relates to calorimetry of spontaneous chemical reactions of pyrolysis oil.

Background of the Invention

Some substances, such as bio-oils, undergo spontaneous chemical reactions, such as exothermic chemical reactions. Because of these reactions, the temperature of the substance changes, at least if the heat is not conducted away (for exothermic reactions) of brought to the substance (endothermic reactions). Moreover, such substances may have a critical temperature, wherein the state of the substance changes. For example, the viscosity may rapidly increase above a critical temperature, or below another critical temperature. The change of state may be a result of chemical reactions. The reactions are not necessarily reversible. Thus, bio-oil stored in a thermally insulated container may spontaneously undergo reactions which essentially solidify the bio-oil, making it difficult to take the solidified bio-oil out of the container.
To overcome the problem, reasonable temperature control should be provided to the bio-oil at least initially, when the spontaneous reactions take place. After storing for a sufficient period of time, the spontaneous reactions become so slow, that no temperature control is needed. However, for determining the sufficient period of time, the heat produced by said spontaneous reactions needs to be known or measured. The reactions are typically very slow. Alternatively, the vessel for storing bio-oil may be equipped with heaters or coolers. However, these heaters or coolers need to be sufficiently dimensioned for the heat produced (or used) in the spontaneous reactions.
Calorimetry refers to measuring changes of state variables of a body for the purpose of deriving the heat transfer associated with changes of its state due for example to chemical reactions, physical changes, or phase transitions under specified constraints. Calorimetry is performed with a calorimeter.
In the art, e.g. differential scanning calorimeters and reaction calorimeters are known. Most calorimeters work in such a way that the temperature of the substance is adjusted to equal the temperature of the environment. This is done in order to minimize the heat flow from the substance to the environment due to thermal conduction. Moreover, heat transfer from the substance to the environment is measured to determine the rate of heat release (or heat generated) at said temperature. Still further, the temperature of the environment may be adjustable to measure these values as function of temperature. Such equipment is typically reasonable expensive.
When the chemical reaction is slow, the sample needs to be measured for a long time. Moreover, for accurate measurements and reasonable statistics, several samples should be measured. In typical calorimeters multiple measurements can only be made subsequently. Therefore, extremely long experiments are required. As the equipment is expensive, the measurements become increasingly expensive.
Calorimeters and calorimetry have been disclosed in the documents DE4414082 A1 and US4783174 A.
The former document discloses an isoperibol calorimeter for measuring the reaction heats of chemical reactions and for determining the time of the reaction start by measuring the temperatures in a reaction vessel and a surrounding thermal bath and recording them on a data-acquisition device. The latter document discloses a continuously operating non-adiabatic, differential calorimeter, for measuring the heat capacity of small samples.

Summary of the Invention

The purpose of the invention is to present a method, by which the heat or rate of heat release of a slow reaction of a substance or substances can be measured. Moreover, the method is easily parallelizable, i.e. several samples can be measured simultaneously. Still further, the equipment needed for the method is reasonable simple, in order to avoid significant investment costs.
The method can be used e.g. to determine effective kinetic reaction parameters of the aforementioned spontaneous reactions of bio-oil. Thus the method enables the determination of the aforementioned sufficient period of time.
The method is described in the independent claim 1. A corresponding computer program is disclosed in the independent claim 14.
Some embodiments are presented in the dependent claims 2 to 13 and 15.

Description of the Drawings

Fig. 1    shows a setup for measuring heat produced by a slow reaction of a substance or substances from one sample,
Fig. 2    shows the results from measurements using the setup of Fig. 1,
Fig. 3    shows a setup for measuring heat produced by a slow reaction of a substance or substances using four samples,
Fig. 4    shows the results from measurements using the setup of Fig. 3, and
Figs. 5a-5d    show examples of first and second primary and secondary periods of time.

Detailed Description of the Embodiments

The purpose of the invention is to present a method, by which the heat or rate of heat release of a slow reaction of a substance or substances can be measured. A slow exothermic reaction has a rate of heat release less than 0.5 W/kg. The reaction heat, i.e. the time integral of the rate of heat release, depends on how long the reaction goes on. If, as an example, the specific rate of heat release, $q_1(t)/(\rho_1 V_1)$, is initially 0.5 W/kg and speed of the reaction exponentially decreases to 1% in two weeks, a total of 130 kJ/kg of heat will be produced. Suppose the heat capacity is 2 J/gK, as is typical to a vegetable oil, in adiabatic conditions the temperature will increase by 65 °C in said two weeks. The temperature increase (or decrease for endothermic reactions) in adiabatic conditions due to the reaction may be at most 2 °C/h. A slow endothermic reaction has a rate of heat consumption less than 0.5 W/kg.

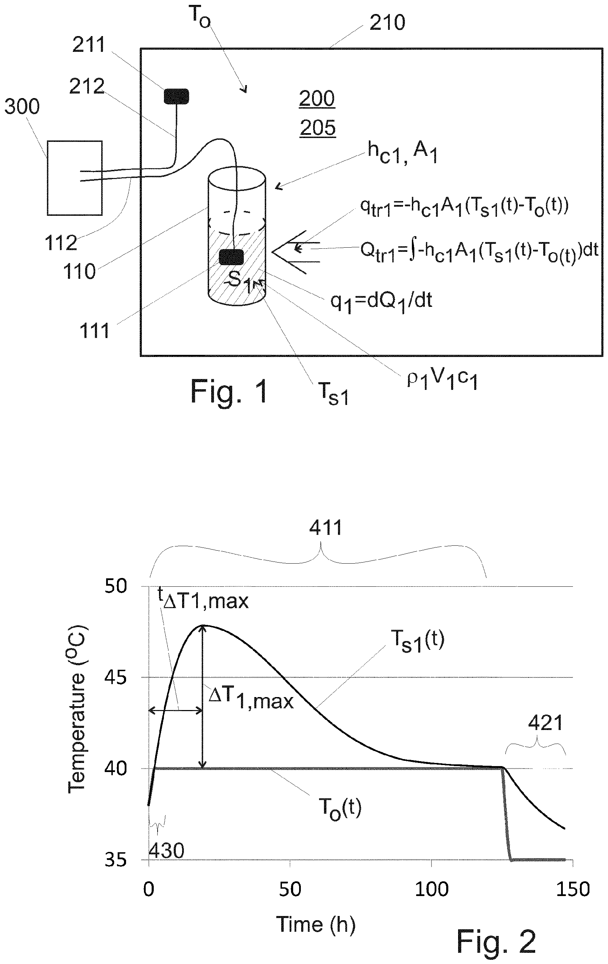

Referring to Fig. 1, in an embodiment of the method, the substance $S_1$ or substances, which undergoes spontaneous chemical reactions is arranged in a first thermally insulating container 110.

The container 110 is arranged in an environment. In the following, during a first primary period of time 411, the container 110 is arranged in a first environment 200 and during a first secondary period of time 421, the container 110 is arranged in a second environment 205. The first and the second environments 200, 205 may be e.g. the environment inside an oven 210.

The arrangement of Fig. 1 comprises a first temperature sensor 111 configured to sense the temperature $T_{s1}$ of the substance $S_1$ (or substances $S_1$) arranged in the first thermally insulated container 110. The arrangement further comprises a first wire 112 for sending a measurement signal from the first temperature sensor 111 to a control unit 300. The measurements are made as function of time t, whereby a temporal temperature profile $T_{s1}(t)$ is measured (see Fig. 2).

The arrangement of Fig. 1 further comprises a zeroth temperature sensor 211 configured to sense the temperature $T_o$ of the first environment 200 (such as the environment inside an oven 210). The arrangement further comprises a zeroth wire 212 for sending a measurement signal from the zeroth temperature sensor 211 to the control unit 300.

One aspect of the embodiment of the method is that there are no special quality requirements for the container 110. Quality here refers to the deviations of properties between substantially similar containers (110, 120, 130, 140), not to absolute values of the properties. Absolute values will be discussed later.

As is conventional in the art, the amount of heat (or the rate thereof) transferred from the environment 200, 205 to the first thermally insulated container 110 during measurements has the opposite (i.e. negative) value compared to the amount heat (or rate thereof) transferred from the first thermally insulated container 110 to its environment 200, 205 during measurements. It is noted that with these definitions, for exothermic reactions, the heat transferred to the first thermally insulated container 110 from the first environment 200 is typically negative.

Even if the container 110 is thermally insulated, the slow chemical reactions imply a long measurement time. Thus, the heat conducted between the substance $S_1$ and the first environment 200 cannot be neglected, at least when the temperature $T_{s1}(t)$ of the substance $S_1$ differs from the temperature $T_o(t)$ of the environment 200.

Therefore, and because of the aforementioned low quality, in an embodiment of the method, the total amount of heat or the instantaneous rate of heat transferred to the first thermally insulated container 110 from the first environment 200 during measurements is determined. This is done, because the deviations of the properties of the container 110 ensures that one cannot rely on values taken from a catalogue.

Referring to Fig. 2, using these components, in the method, the temporal temperature profile $T_{s1}(t)$ of the substance $S_1$ or the substances arranged in the first thermally insulating container 110 is measured during a first primary period of time 411. Thus, the measured profile is also a profile for the first primary period of time 411. Moreover, the temporal temperature profile $T_o(t)$ of the first environment 200 is determined for the first primary period of time 411. Measuring a temporal profile (of the substance $S_1$) means measuring multiple values of the temperature at different instances of time. The number of said multiple values of the profile may be e.g. at least five, preferably more than ten or more than twenty. Determining the temperature profile of the environment 200, 205 may be done also based on e.g. a known temperature profile of the environment 200, 205, e.g. when an oven 210 is arranged to control the temperature of the first environment 200 according to a given profile $T_o(t)$. Thus, the user of such an oven does not have to measure the profile, because he can rely on that the temperature follows the given profile. The temperature profile $T_{s1}(t)$ of the substance $S_1$ in the container 110 is governed by the heat balance equation:

$$\left(\rho_1 V_1 c_1 + C_{c,1,eff}\right)\frac{dT_{s1}(t)}{dt} = -h_{c1}A_1(T_{s1}(t) - T_o(t)) + q_1(T_{s1}(t), x_1), \qquad (1)$$

wherein $\rho_1$ is the density of the substance $S_1$ (in kg/m$^3$), $V_1$ is the volume of the substance $S_1$ (in m$^3$), and $c_1$ is the heat capacity of the substance $S_1$ (in Jkg$^{-1}$K$^{-1}$). In a typical experiment, the heat capacity in constant pressure $c_{1,p}$ can be used as the heat capacity $c_1$, as the pressure in the thermally insulated container 110 can become equal with the pressure of the first environment e.g. via the hole through which the wire 112 has been inserted into the container 110. Provided that the container 110 is gas-tight, the heat capacity $c_{1,v}$ in a constant volume may be more appropriate. The right hand side of Eq. (1) shows how the heat is conveyed to or from the container 110.

The term $-h_{c1}A_1(T_{s1}(t)-T_o(t))$ describes the heat transfer rate to the container, and $q_1$ is the heat generated or needed in the substance $S_1$ because of the slow chemical reaction.

$C_{c,1,eff}$ is the effective heat capacity of the first thermally insulating container 110. When the substance $S_1$ and the environment are at different temperatures, the outer surface of the container 110 has a temperature reasonable close to the temperature of the environment, and the inner surface of the container 110 has a temperature reasonable close to the temperature of the substance. In between, the temperature profile inside the walls of the container 110 depends on the insulating properties and/or structure of the container. Thus, $C_{c,1,eff}$ is somewhat smaller than the heat capacity of the container 110. Preferably, the heat capacity of the container 110 is low compared to the heat capacity of the substance. In an embodiment, the heat capacity (or the effective heat capacity) of the container 110 is at most 15 % of $\rho_1 V_1 c_1$. Moreover, conventional thermally insulating containers, such as vacuum flasks, have reasonable low heat capacity, at least, when the substance $S_1$ (or a part thereof) is in liquid form. Thus, in an embodiment, a part of the substance or the substances in the container 110 are in liquid form. Moreover, the heat capacity of the container 110 needs not be measured. In an embodiment, heat capacity of the first thermally insulating container 110 is not measured.

It is noted that from the left hand side of Eq. 1, the effective heat capacity of the thermally insulated container 110 can be neglected, if it is small compared to the total heat capacity $\rho_1 V_1 c_1$ of the substance $S_1$. The thermally insulating container 110 may be e.g. a vacuum flask (also known as Thermos®).

Alternatively, the thermally insulating container may be e.g. a polystyrene box or a polyurethane box. If needed, a liquid proof pouch can be used inside such a box for preventing the substance from diffusing into the walls of the box.

On the right hand side of Eq. 1, the term $-h_{c1}A_1(T_{s1}(t)-T_o(t))$, which is later denoted by $q_{tr1}$, describes the rate of heat transfer to the container 110 from the first environment due to temperature difference in between the substance $S_1$ and the first environment 200. Positive values, which occur when $T_{s1}<T_o$, indicate heat transfer to the container 110. For exothermic reactions, typically $T_{s1}>T_o$ (see Fig. 2), whereby heat is transferred away from the substance $S_1$. Here the term $h_{c1}$ is the heat transfer coefficient of the container 110 in the first environment 200 (in Wm$^{-2}$K$^{-1}$) and $A_1$ is the area (in m$^2$) of the container 110. The product $A_1 h_{c1}$ will be referred to as the total heat transfer coefficient.

It should be noted that $h_{c1}$ depends not only on the container 110, but also on the first environment 200. For example, if the first environment 200 is a liquid bath, such as a water bath, $h_{c1}$ may be higher than in the case where the first environment e.g. consists of air. Thus, the product $A_1 h_{c1}$ can be referred to as the total heat transfer coefficient to the container 110 from the environment 200 (or 205). In principle, $h_{c1}$ can depend on temperature. Moreover, the area $A_1$ only negligibly depends on temperature due to thermal expansion. It is also noted that the term $A_1 h_{c1}$ takes into account also heat transfer by radiation. Radiative heat transfer may occur e.g. between inner and outer surfaces of a vacuum flask (i.e. the container 110) and/or from the outer surface of the container 110 to the environment (200, 205). It is also noted, that in the long run, heat transferred to the container 110 from the environment (200, 205) equals the heat transferred to the substance $S_1$ from the environment (200, 205); at least with reasonably accuracy when the heat capacity of the container is low.

In general, the heat transfer $q_{tr1}$ between the container 110 and the environment 200 is a problematic issue. Therefore, in some calorimetric measurements, the temperature of the environment is made to follow the temperature of the substance, whereby the process may be adiabatic. However, in particular, when more than one containers 110, 120, 130, 140 are used, it is impossible to make the temperature of the environment to follow the temperature inside both (or all) the containers (110, 120, 130, 140), since the temperatures inside the containers are different. A purpose of the invention is to take into account the heat transfer $q_{tr1}$ from the environment (200, 205) to the container 110 (or vice versa, as discussed above).

On the right hand side of Eq. 1, the term $q_1(T_{s1}(t), x_1)$ is the rate of heat release (in W) of the reactions of the substance $S_1$. $q_1(T_{s1}(t), x_1)$ may be positive (exothermic reactions) or negative (endothermic reactions). This term may alternatively be written as $q_1(t)$, since both the temperature of the substance $T_{s1}(t)$ and the concentration $x_1$ depend on

time t. The rate of heat release $q_1$ is related to the heat produced by the reaction $Q_1$ by $q_1=dQ_1/dt$ as discussed above. Moreover, the reaction heat is the time integral of the rate of heat release, when integrated to the end of the reactions.

In an embodiment, the reaction is exothermic, whereby the rate of heat release $q_1$ is positive.

The speed of the reactions in the substance $S_1$ in the container 110 depends on one hand on the concentration, and on the other hand on temperature via the Arrhenius equation. Moreover, the rate of heat release $q_1$ depends on the speed via the reaction energy. Thus, the rate of heat release is governed by

$$q_1(T_{s1}(t), x_1) = A_{f1} e^{-E_{a1}/(RT_{s1}(t))} x_1 \Delta H_1, \tag{2}$$

where $A_{f1}$ is the pre-exponential factor of the reaction (in 1/s), $E_{a1}$ is the activation energy of the reaction (in J/mol), R is the gas constant (8.31 $JK^{-1}mol^{-1}$), $x_1$ is the (proportional) concentration (no units), and $\Delta H_1$ is the energy of the reaction (in J). The concentration $x_1$ may be assumed to initially equal one. The concentration decreases as the reaction proceeds.

The change of the concentration is governed by

$$\frac{dx_1}{dt} = -x_1 A_{f1} e^{-E_{a1}/(RT_{s1}(t))} . \tag{3}$$

Thus, the concentration tends towards zero in the long run.

It is noted that in the substance $S_1$, multiple different chemical reactions may take place at the same time. Eqns. (2) and (3) apply, strictly speaking, to only one reaction. However, these equations may be seen as an approximate equation describing, on the average, the heat release rate of the combination of these multiple different chemical reactions. The subscript 1 is used in the equations 1-3, as these values refer to the substance $S_1$ in the first container 110. As will be discussed, multiple containers may be used.

It is noted that the speed of the reactions may be limited also by the mixing of the constituents. Convection of the substances $S_1$ in the container can be improved by agitation, such as shaking the container 110. In an embodiment, the substance $S_1$ is agitated during at least a part of the primary period 411.

Figure 2 shows schematically an example of a measured temperature profile, where spontaneous exothermic reactions undergo in a bio-oil (specifically pyrolysis oil) during a first primary period of time 411. The temporal profile of the temperature $T_{s1}(t)$ of the substance $S_1$ during the first primary period 411 is depicted in the Figure. Because the container 110 is thermally insulated and exothermic reactions take place, the temperature of the substance $S_1$ increases initially. For a short initial period, i.e. during an initial stabilization period 430, the temperature increases also by conduction, as, in the beginning, the temperature of the substance, $T_{s1}(0)$, in this case has been less than the temperature of the first environment, $T_o(0)$. Naturally the temperatures $T_{s1}(0)$ and $T_o(0)$ may also be equal.

In the method it is preferable, that the container 110 is sufficiently well thermally insulated. The insulation should be so good that the rate of heat release generated in the (exothermic) process exceeds the heat conducted from the substance due to temperature difference. More precisely, and also for endothermic reactions, preferably

$$|q_1| > |h_{c1} A_1 \Delta T_{cr}|, \tag{4}$$

wherein $|q_1|$ denotes the absolute value of $q_1$. Equation (4) should apply for at least a suitable critical temperature difference $\Delta T_{cr}$ and at least initially, when the concentration $x_1$ is large (see Eqns. 1-3). A suitable critical temperature difference, in case of bio-oils, have been found to be e.g. at least 5 °C.

Based on these lines, the skilled person can select a suitably well insulating container 110 (i.e. having a low value of $A_1 h_{c1}$ and thus also $h_{c1}$); at least after some experimentation with the case at hand.

For some period of time, in Fig. 2 for about 20 hours, the temperature increases, as the container 110 is insulating and exothermic reactions occur. The converse is true for endothermic reactions. However, after such period, the concentration $x_1$ has decreased (see Eq. 3) to such a low level, that the heat transfer $q_{tr1}$ through the walls of the container 110 exceed the heat $q_1$ produced in the substance $S_1$, whereby the temperature $T_{s1}(t)$ no longer increases. Thereafter, the temperature turns decreasing, since the conduction by $q_{tr1}$ overcomes $q_1$, because $x_1$ has become small (see Eqns. 2 and 3).

If the total heat transfer coefficient $A_1 h_{c1}$ is known, the heat transfer rate $q_{tr1}$ transferred to the first container 110 from the first environment 200 during the first primary period of time 411 can be calculated after the temporal temperature profiles $T_{s1}(t)$ and $T_o(t)$ have been measured. In an embodiment, a time constant is measured, from which the rate of heat release $q_1$ and/or the total heat transfer coefficient $A_1 h_{c1}$ can be calculated, as will be described.

Provided that the total heat capacity $\rho_1 V_1 c_1$ and the total heat transfer coefficient $A_1 h_{c1}$ are known, the rate of heat release $q_1$ as function of time can be determined simply from Eq. (1) by rearranging terms. Thus, if needed, also the reaction heat $Q_1$, which is the time integral $\int q_1$, can be determined.

As shown in Fig. 2, during at least an instance of time comprised by the first primary period of time 411 the temperature $T_{s1}$ of the substance $S_1$ is different from the temperature $T_o$ of the first environment by at least 1 °C. Preferably, and as indicated in Fig. 2, during at least 10 % of the first primary period of time 411, the temperature $T_{s1}$ of the substance S is different from the temperature $T_o$ of the first environment by at least 1 °C. Moreover, the temperature $T_{s1}$ may be different from the temperature $T_o$ for at least 10 hours during the first period 411 and by at least 1 °C. This typically happens, if the container 110 is sufficiently well insulated. However, as will become clear, by increasing the length of the first period 411, during a substantial part of the period 411, the temperature difference between the substance and the environment may be small.

Moreover, as shown in Fig. 2, typically the reaction is so slow that the first primary period of time 411 spans at least 50 hours. The first primary period of time may span at least 100 hours, at least 200 hours, or even more. In Fig. 2, the period 411 spans 125 hours. Preferably, the temperature $T_o(t)$ of the first environment 200 is constant or substantially constant during the first primary period of time 411. The temperature $T_o(t)$ of the first environment 200 may be e.g. in the range $\langle T_o \rangle \pm 10$ °C during the first primary period of time 411, wherein $\langle T_o \rangle$ is the average temperature of the first environment 200 during the first primary period of time 411 (average over time). More preferably, $T_o(t)$ is in the range $\langle T_o \rangle \pm 3$ °C during the first primary period of time 411. This simplifies the calculations.

It has been noticed that the total heat transfer coefficient $h_{c1} A_1$ can be determined after at least most of the reactions have occurred from a time constant of temperature relaxation. Referring to Fig. 2, after the first primary period of time 411, the container 110 is introduced in another environment 205 or the same environment 205 with a different temperature. More specifically, after the rate of heat release of the reaction, as determined by Eq. 2, has sufficiently decreased, the container 110 is introduced in a second environment 205, of which temperature (on the average) is different from the average temperature of the environment 200 surrounding the container 110 during the first primary period of time 411. For example, the container 110 may be held in the same temperature controlled oven 210, wherein only the temperature of the space inside the oven 210 is changed, resulting in a second environment 205. However, the container 110 could be transferred e.g. into another oven surrounding a second environment 205.

After the rate of heat release of the reaction has sufficiently decreased, the temporal temperature profile $T_{s1}(t)$ of the substance or the substances, still being arranged in the first thermally insulating container 110, which now is surrounded by a second environment 205, is measured at a first secondary period of time 421 and as function of time t. Moreover, for the first secondary period of time 421, the temporal temperature profile $T_o(t)$ of the second environment 205 is determined. It can be determined from the reading of the oven 210 or by measuring, as discussed above for the first primary period 411 and the profile $T_o(t)$.

When the concentration $x_1$ has sufficiently decreased, the rate of heat release $q_1$ may be essentially zero, or at least $|q_1(t)|$ is much less than $|h_{c1} A_1 \Delta T_1(t)|$; where $\Delta T_1(t) = T_{s1}(t) - T_o(t)$). Moreover, in an embodiment, the effective heat capacity of the container 110 is negligibly small. Therefore, the temperature of the substance, during the first secondary period 421, is governed by (see Eq. 1, when $q_1 = 0$ and $C_{c,1,eff=0}$):

$$\frac{dT_{s1}(t)}{dt} = \frac{-h_{c1} A_1}{\rho_1 V_1 c_1}(T_{s1}(t) - T_o(t)). \tag{5}$$

From this equation, the total heat transfer coefficient $A_1 h_{c1}$ can be solved, when the temporal temperature profiles $T_{s1}(t)$ and $T_o(t)$ are known, in addition to the total heat capacity $\rho_1 V_1 c_1$. This can be done e.g. by fitting the measured data to Eq. 5. For example, if $T_o(t)$ is constant during the first secondary period 421, the solution to Eq. 5 has an exponentially decaying form $\Delta T_1(t) = \exp(-t \times h_{c1} A_1/(\rho_1 V_1 c_1))$ during the period 421. Thus, $\log(\Delta T_1(t))$ depends linearly on time, and the time constant can be found by calculating the slope.

Because of this measurement, preferably, the temperature $T_o(t)$ of the second environment 205 is constant or substantially constant during the first secondary period of time 421. The temperature $T_o(t)$ of the second environment 205 may be e.g. in the range $\langle T_o \rangle \pm 10$°C during the first secondary period of time 421, wherein $\langle T_o \rangle$ is the average temperature of the second environment 205 during the first secondary period of time 421 (average over time). More preferably, $T_o(t)$ is in the range $\langle T_o \rangle \pm 3$ °C during the first secondary period of time 421. This simplifies the calculations.

After the total heat transfer coefficient $A_1 h_{c1}$ has been solved, the heat transfer rate $q_{tr1}$ or the heat $Q_{tr1}$ transferred to the first thermally insulated container 110 from the first environment 200 during the first primary period of time can be calculated from Eq. 1. Thereby the heat release rate $q_1$ can be calculated.

Alternatively, one can use Eq. 5 and the temporal temperature profile during the secondary period of time to determine the time constant $(A_1 h_{c1})/(\rho_1 V_1 c_1)$ for temperature decay. By rearranging terms and dividing Eq. 1 with the total heat capacity $\rho_1 V_1 c_1$, one may write:

$$\frac{q_1(T_{s1}(t),x)}{\rho_1 V_1 c_1} = \frac{dT_{s1}(t)}{dt} + \frac{h_{c1}A_1}{\rho_1 V_1 c_1}(T_{s1}(t) - T_o(t)). \qquad (6)$$

In this way, the term $q_1/(\rho_1 V_1 c_1)$ can easily be calculated from the measured temperature data. This can be done, because $T_{s1}(t)$ is measured and $T_o(t)$ is determined during the primary period 411, and the time constant $(A_1 h_{c1})/(\rho_1 V_1 c_1)$ can be determined from the measurements at the secondary period 421, as discussed above. Moreover, the time derivative can be numerically calculated from measurements. In an embodiment, information indicative of the total heat capacity $\rho_1 V_1 c_1$ of the substance $S_1$ in the first thermally insulating container 110 is received. Part of this information may be received from a book or from the Internet, and another part may be measured. Alternatively, the heat capacity (total or specific) can be measured. Thus, the rate of heat release, $q_1$, can be calculated from $q_1/(\rho_1 V_1 c_1)$. Alternatively or in addition, the total heat transfer coefficient $h_{c1}A_1$ can be calculated from the time constant. Typically $h_{c1}A_1$ is specific to the container 110 and to the environment 200, 205.

Moreover, as evidenced by Eq. 6 and the time constant, an embodiment comprises determining - using the temperature profiles $T_{s1}(t)$ and $T_o(t)$ of the substance $S_1$ and second environment 205 during the first secondary period 421 - information that is, in combination with the information indicative of the total heat capacity and the temperature profiles of the substance and the environment during the first primary period of time 411, indicative of heat transfer between the first thermally insulating container 110 and its environment 200 during the first primary period of time 411.

One term affecting this time constant is the ratio $V_1/A_1$, which in general increases when the size of the first container 110 increases. Thus, preferably the volume $V_1$ is less than 200 litres. Also preferably, the ratio $V_1/A_1$ is less than 11 cm. Alternatively, $V_1/A_1$ may be at most 6 cm. Alternatively or in addition, $V_1$ may be at most 100 litres. For a larger container 110, the measurements may become very slow and/or the temperature increase in the container 110 may become excessive.

Moreover, rate of heat release $q_1$ of the reactions depends on the amount of reacting material, which is proportional to the volume $V_1$ of the substance in the first container 110. However, the heat conduction from the first container 110 is proportional to the area $A_1$ of the container 110. Thus, the requirement of Eq. 4 can also be affected by a proper ratio of $V_1/A_1$. In an embodiment, the ratio $V_1/A_1$ is at least 3 mm. Also preferably, the volume $V_1$ is at least 0.1 litres. Alternatively, $V_1/A_1$ may be at least 5 mm. Alternatively or in addition, $V_1$ may be at least 0.2 litres.

In Eqns. 5 and 6 it has been assumed that the heat capacity of the container is negligible. The ratio $V_1/A_1$ affects also the ratio of the heat capacity of the container to the heat capacity of the sample. Also for this reason, the ratio $V_1/A_1$ should not be too small. Specific examples were given above.

Furthermore, in the Eqns. 5 and 6 (and also elsewhere), an underlying assumption is that the temperature of the substance $S_1$ in the container 110 is constant, or at least substantially constant. Thus, unless agitation is used during measurements, the container should be reasonable small. Or more specifically, the ratio $V_1/A_1$ should be reasonably small. Specific examples were given above.

The point of time, when the speed of the reactions have sufficiently decreased, may be determined e.g. from the temperature profile of the substance, i.e. $T_{s1}(t)$. Referring to Fig. 2, the temperature profile $T_{s1}(t)$ may have an initial stabilization period 430, wherein both the conduction from the first environment 200 to the container 110 and the spontaneous reactions in the container 110 affect the temperature of the substance $S_1$ in the same way; i.e. both increase the temperature in Fig. 2. This period lasts to the point of time, wherein the absolute value of the difference between the temperature of the substance $T_{s1}(t)$ and the temperature first environment $T_o(t)$ has its minimum value, i.e. until to that point t of time, wherein $|T_{s1}(t)-T_o(t)|$ has its minimum value. In Fig. 2 this occurs at around t = 2 h.

Thereafter, the temporal temperature profile of the substance or the substances $T_{s1}(t)$ is (at least mainly) caused by said reaction or reactions of the substance or substances in the first thermally insulated container 110.

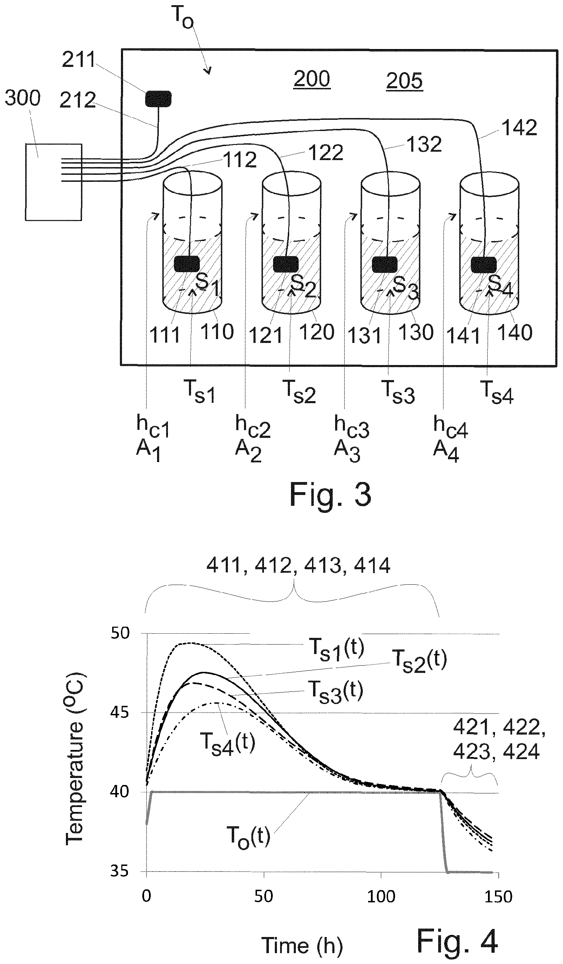

With reference to Figs. 2 and 4, the concentration $x_1$ can be said to have sufficiently decreased, e.g. when the absolute value of the temperature difference between the substance and the environment, i.e. $|T_{s1}(t)-T_o(t)|$ has decreased to one fifth (i.e. 20 %) of its maximum value $\Delta T_{1,max}=max|T_{s1}(t)-T_o(t)|$ (see Fig. 2). Alternatively, the temperature difference may decrease to 10 % or 5 % of its maximum during the first primary period 411. When two containers are used, this condition applies preferably to both samples of both containers 110 and 120. When multiple containers are used, this condition applies preferably to all samples of such containers 110, 120, 130, 140 that are used for measurements.

Alternatively or in addition, one may determine the time $t_{\Delta T1,max}$ when the maximum absolute temperature difference $\Delta T_{1,max}$ occurs (see Fig. 2). This time is measured from the start of the corresponding first primary period of time (411 in Fig. 2). The reactions may be sufficiently slow, i.e. the concentration $x_1$ can be said to have sufficiently decreased, when sufficient time has passed after the occurrence of the maximum temperature difference; optionally in addition to sufficiently small $|T_{s1}(t)-T_o(t)|$. For example, the first primary period 411 may last for at least $5 \times t_{\Delta T1,max}$, at least $10 \times t_{\Delta T1,max}$ or at least $20 \times t_{\Delta T1,max}$.

It is noted that preferably the first primary period of time 411 is determined to have ended relatively soon after the speed of the reactions have slowed down sufficiently. This reduces the measurement time.

As discussed above, the substance $S_1$ is at different temperature than the environment 200 for at least some time during the first primary period 411, whereby heat transfer must occur between the substance and the environment 200. As heat transfer occurs, the thermal resistance of the interface between the container and the environment also plays a role in heat transfer. Therefore, the second environment 205 (i.e. the environment of the container 110 during the first secondary period of time 421) is selected in such a way that the medium surrounding the container 110 is in the same state (liquid or gas) during the primary and secondary periods 411, 421. E.g. if a liquid bath is used as the first environment 200 during the first primary period 411, a liquid bath is used also as the second environment 205 during the first secondary period 421. Moreover, if the first environment 200 during the first primary period 411 is gaseous, a gaseous second environment 205 is used also during the first secondary period 421. Preferably, the medium surrounding the container 110 is the same during the primary and secondary periods 411, 421. Preferably, the environments 200, 205 are such that air surrounds the thermally insulating container(s) in both environments 200 and 205. This simplifies the equipment compared to e.g. a water bath.

Preferably, as indicated in Fig. 2, the first secondary period of time 421 starts soon after the first primary period 411 of time has ended. The first secondary period of time 421 may start at the moment the first primary period of time 411 ends. In an embodiment, the first secondary period of time 421 may start at most one hour after the first primary period of time 411 ends. For example, if the container 110 is moved to another oven, some time may elapse in between the periods 411, 421. However, preferably the time is relatively short to increase the accuracy of measurements and decrease the measurement time. Moreover, moving the container to another environment may expose the container to an environment with an unknown temperature. This may deteriorate the results.

The average temperature of the second environment 205 (i.e. the average of $T_o(t)$ during the first secondary period of time 421) is different from the average temperature of the first environment 200 (i.e. the average of $T_o(t)$ during the first primary period of time 411). This has the effect that the rate of heat release $q_1$ and heat transfer rate $-A_1 h_{c1} \Delta T_1(t)$ of the reaction and conduction, respectively, can more easily be distinguished. (Also here $\Delta T_1(t) = T_{s1}(t) - T_o(t)$).

Moreover, preferably, the average temperature of the second environment 205 (i.e. the average of $T_o(t)$ during the first secondary period of time 421) is less than the average temperature of the first environment 200 (i.e. the average of $T_o(t)$ during the first primary period of time 411). This has the effect that by Eq. (2), the reactions even further slow down, whereby the assumption of Eq. 5 that $q_1$ is essentially zero applies to an even further degree of accuracy. Preferably, the average temperature of the second environment 205 (i.e. the average of $T_o(t)$ during the first secondary period of time 421) is less than the average temperature of the first environment 200 (i.e. the average of $T_o(t)$ during the first primary period of time 411) by at least 2 °C or at least 4 °C. This temperature difference should not be too high, since then the possible temperature dependence of $h_{c1}$ may affect the results. Therefore, preferably, the average temperature of the second environment 205 during the first secondary period of time 421 is less than the average temperature of the first environment 200 during the first primary period of time 411 by at most 50 °C, at most 20 °C, or most 10 °C. Moreover, heat transfer between the container and the environment can be assumed linear and can be described with the coefficient $h_{c1}$, when the absolute value of temperature difference, $|(T_{s1}(t) - T_o(t))|$, is reasonably small. Preferably $|(T_{s1}(t) - T_o(t))|$ is at most 100 °C.

As indicated above, in an embodiment, the first thermally insulated container 110 is arranged in the same temperature-controlled environment during the first primary period of time 411, the first secondary period of time 421, and - provided that there is a time in between these periods 411 and 421 - also in between these periods (411, 421). As indicated above, the first secondary period of time 421 is later than the first primary period of time 411. This has the effect that spontaneous reactions during the secondary period 421 are sufficiently slow for the determination of the heat transfer coefficient in the described manner.

The controllable temperature of the environment (200, 205) has the technical effect that the spontaneous reactions can be accelerated or decelerated by changing the temperature of the first environment 200 or the second environment 205, as evidenced by Eq. 2. Thus, the measurement time can be decreased to some degree by increasing the temperature of the first environment with respect to e.g. room temperature. In an embodiment of the method, the temperature of the first environment is controlled. However, it is well known that too much acceleration results in reactions that would not at all take place in normal storage conditions. Therefore, the temperature during the period 411 is preferably not too high. In an embodiment of the method, the temperature of the first environment 200 is in between 20 °C and 100 °C during the first primary period 411.

Moreover, Eq. 1 takes the radiative heat transfer (e.g. between the surfaces of a vacuum flask), which is known to be proportional to $T^4$, wherein T is the temperature in Kelvin, into account only through the term $h_{c1}$, which is assumed independent of temperature. As radiative heat transfer increases rapidly with temperature, both the absolute value temperature difference $|(T_{s1}(t) - T_o(t))|$ and the temperatures themselves should be small. Thus, in addition to the aforementioned restrictions, preferably the temperatures $T_{s1}(t)$ and $T_o(t)$, and absolute difference $|(T_{s1}(t) - T_o(t))|$ during the first primary period of time 411 and first secondary period of time 421 are all at most 200 °C. This can apply at all times during said periods (411, 421), or for at least half of the corresponding period (411, 421). In this way, one may reasonably accurately assume that the radiative heat transfer between the first thermally insulated container 110 and the environment 200 is proportional to the temperature difference between the first thermally insulated container 110 and the environment 200. This may apply irrespective of the liquid that is measured.

As for accelerating the experiments, it is noted that even if Eqns. 2 and 3 may be seen as an average of several different reactions, the heat release rate and the measurement principles described by Eqns. 1, 5, and 6 do not in any way depend on the accuracy of the approximations in Eqns. 2 and 3. For the method, it suffices that the reactions somehow slow down sufficiently during the first primary period of time 411. However, since the equations 2 and 3 may be seen as an average over multiple reactions, acceleration by increasing temperature does not necessarily follow these equations.

Because the arrangement does not comprise a heat flow sensor, the equipment is reasonable simple and cheap. When the method is parallelized, several more or less similar containers 110 can be used simultaneously. However, as the containers are not identical, the heat conducted between the environment and the containers should be detected individually. The principles presented above apply for each container.

Figure 3 shows an arrangement for measuring four samples simultaneously. In Fig. 3, some substance or some substances $S_1$, $S_2$, $S_3$, and $S_4$ are arranged in the first, second, third and fourth thermally insulating containers 110, 120, 130, and 140, respectively. The substance in the other containers 120, 130, 140 may be the same substance as the one in the first container 110, or some other substance or some other substances may be measured and arranged in the other containers.

The first, a second, a third and a fourth temperature sensors 111, 121, 131, and 141 are arranged to measure the temporal temperature profiles $T_{s1}(t)$, $T_{s2}(t)$, $T_{s3}(t)$, $T_{s4}(t)$ of said substance(s) arranged in the first, second, third and fourth thermally insulating containers 110, 120, 130, and 140, respectively.

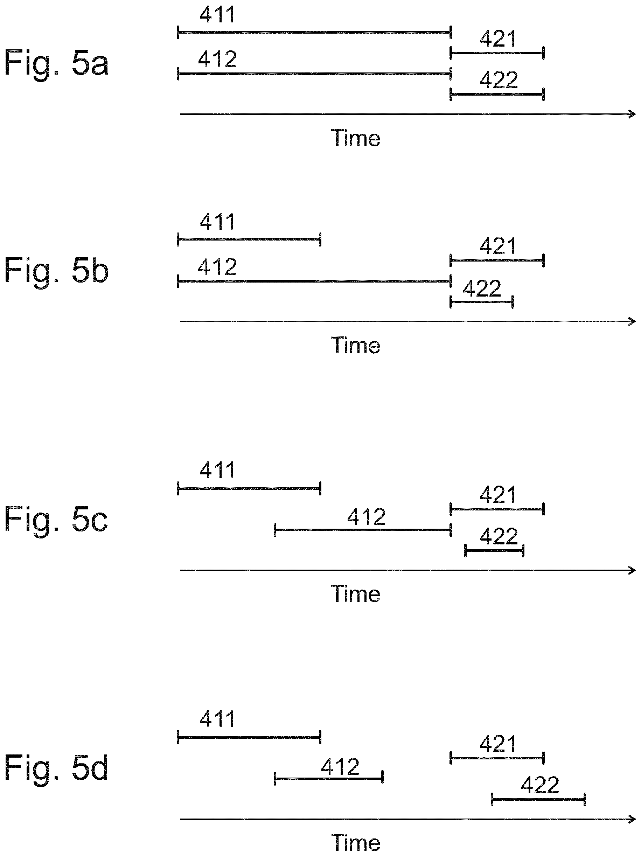

With reference to Figs. 4 and 5a-5d, the temporal temperature profile $T_{s1}(t)$ of the substance $S_1$ of the first container 110 is measured during the first primary period of time 411. The temporal temperature profile $T_{s2}(t)$ of the substance $S_2$ of the second container 120 is measured during a second primary period of time 412. The temporal temperature profile $T_{s3}(t)$ of the substance $S_3$ of the third container 130 is measured during a third primary period of time 413. The temporal temperature profile $T_{s4}(t)$ of the substance $S_4$ of the fourth container 140 is measured during a fourth primary period of time 414. In order to save time, preferably at least two of the primary periods of time 411, 412, 413, 414 at least partly overlap, as indicated in Figs. 5a-5d. More preferably, all the primary periods of time 411, 412, 413, 414 at least partly overlap, as indicated in Figs. 4 and 5a-5d. Even more preferably, all the primary periods of time 411, 412, 413, 414 are the same, as indicated in Figs. 4 and 5a. For example, in case (at least) two containers 110, 120 are used, preferably, the primary periods of time 411, 412 overlap each other by at least 10 % or at least 50 % of the duration of the shorter of the periods, or more preferably they are the same. In an embodiment, for such a period of time that the first primary period of time 411 and the second primary period of time 412 overlap, the first thermally insulating container 110 and the second thermally insulating container 120 are arranged in the same first environment 200. An embodiment comprises determining, for a primary period of time (411, 421), only one temperature profile $T_0(t)$ for the first environment 200 and measuring, during the primary period of time (411, 412), at least two temperature profiles $T_{s1}(t)$, $T_{s2}(t)$.

Moreover, preferably, the time constants $(A_1 h_{c1})/(\rho_1 V_1 c_1)$, $(A_2 h_{c2})/(\rho_2 V_2 c_2)$, $(A_3 h_{c3})/(\rho_3 V_3 c_3)$, and $(A_4 h_{c4})/(\rho_4 V_4 c_4)$ are measured as indicated above. I.e. they are measured at a time, when one can assume that $q_1$, $q_2$, $q_3$, or $q_4$ (respectively) is zero. If they are measured simultaneously, the primary periods of time, 411, 412, 413, 414 are selected so that all $q_1$, $q_2$, $q_3$, and $q_4$ are essentially zero. The time constant $(A_1 h_{c1})/(\rho_1 V_1 c_1)$ is determined using measurements during the first secondary period of time 421. The time constant $(A_2 h_{c2})/(\rho_2 V_2 c_2)$ is determined using similar measurements during a second secondary period of time 422. The time constant $(A_3 h_{c3})/(\rho_3 V_3 c_3)$ is determined using similar measurements during a third secondary period of time 423. The time constant $(A_4 h_{c4})/(\rho_4 V_4 c_4)$ is determined using similar measurements during a fourth secondary period of time 424. Again, to save time, preferably, at least two of the secondary periods of time 421-424 at least partially overlap, as indicated in Figs. 4 and 5a-5d. More preferably, all the secondary periods of time 421-424 at least partially overlap, as indicated in Figs. 4 and 5a-5d. Still more preferably, all of the secondary periods of time 421-424 are the same, as indicated in Figs. 4 and 5a.

The principles of the method as discussed above apply to each thermally insulating container. It is noted that in particular, if the time constants (see Eqns. 5 and 6 and related discussion) of the containers 110, 120, 130, 140 are different, a shorter secondary period may be sufficient for one container than for another container (see Fig. 5b).

When at least two samples are measured, the substance or the substances or some other substance or some other substances $S_2$ are arranged in at least a second thermally insulating container 120. The method can thus be used for multiple different substances simultaneously, and/or to multiple different samples of the same or substantially same substance. During the second primary period of time 412, the second container 120 is arranged in the same environment as the first container 110. This applies also to the other containers 130, 140, if present, and during the corresponding primary periods 413, 414. During the second primary period of time 412, the temporal temperature profile $T_{s2}(t)$ of the substance or the substances or the other substance or the other substances $S_2$ arranged in the second thermally insulating container 120 is measured. In a similar way, the profiles $T_{s3}(t)$ and $T_{s4}(t)$ of the substances $S_3$ and $S_4$ of the

other containers 130 and 140 may be measured during a third and fourth primary period of time (413, 414).

Moreover, the heat transfer rate (i.e. $q_{tr2}=-h_{c2}A_2(T_{s2}(t)-T_o(t))$) or transferred heat $Q_{tr2}$ (which is the time integral of $q_{tr2}$) to the second thermally insulated container 120 from the first environment 200 during the second primary period of time 412 is determined. This applies also to the further heat transfer rates (i.e. $-h_{c3}A_3(T_{s3}(t)-T_o(t))$ or $-h_{c4}A_4(T_{s4}(t)-T_o(t))$) or further transferred heats (i.e. time integral thereof)) during their primary periods of time (413, 414).

Still further, information indicative of a total heat capacity $\rho_2V_2c_2$ of the substance $S_2$ in the second container 120 is received. As the substance $S_2$ may refer to the same or another substance as the substance $S_1$, an embodiment comprises receiving information indicative of a total heat capacity $\rho_2V_2c_2$ of the substance or the substances or the other substance or the other substances $S_2$ arranged in the second thermally insulating container 120. Herein $\rho_2$, $V_2$, and $c_2$ refer to the density, volume and heat capacity of the second substance $S_2$. The heat capacity in constant pressure $c_{2,p}$ or in constant volume $c_{2,v}$ can be used as the heat capacity $c_2$, depending on experimental setup (pressure-regulated or gas-tight). Moreover, information indicative of the other total heat capacities $\rho_3V_3c_3$ and $\rho_4V_4c_4$ of the substances $S_3$ and $S_4$ in the containers 130, 140 is received, if four samples are measured.

In a similar manner to what has been described in the case for one sample (see Eqns. 5 and 6 in particular), the rate of heat release $q_2$ or the heat $Q_2=\int q_2$ produced by the reaction of the substance or the substances or the other substance or the other substances $S_2$ arranged in the second thermally insulating container 120 is determined using the aforementioned profile $T_{s2}(t)$, the rate of heat release $q_{tr2}$ or heat (time integral thereof)) transferred from the first environment during the second primary period of time 412 to the second container 120, and the total heat capacity $\rho_2V_2c_2$ of the substance $S_2$.

Because

- the substance $S_2$ in the second container 120 is not necessarily identical to the substance $S_1$ in the first container 110, whereby

  ○ the rate of heat release $q_2$ in the second container 120 is not necessarily equal to the rate of heat release $q_1$ in the first container 110 and/or

  ○ the total heat capacity $\rho_2V_2c_2$ of the substance of the second container 120 is not necessarily equal to the total heat capacity $\rho_1V_1c_1$ of the substance of the first container 110 and/or

- the total heat transfer coefficient $h_{c2}A_2$ of the second container 120 in its environment is different from the total heat transfer coefficient $h_{c1}A_1$ of the first container 110 in the environment,

  the temperature changes in a different manner in the containers 110, 120, 130, and 140; in particular in the first and the second container 110, 120.

Obtaining identical containers would require, from the manufacturer thereof, negligible manufacturing tolerances, i.e. infinitely high quality. This would be extremely costly. To achieve a cost effective and parallelizable method, the method must be applicable also in a case, where the containers 110, 120, 130, and 140 are not identical. Therefore, in an embodiment, such containers 110, 120 and/or samples $S_1$, $S_2$ are used that the corresponding time constants $(A_1h_{c1})/(\rho_1V_1c_1)$ and $(A_2h_{c2})/(\rho_2V_2c_2)$ are different. In an embodiment, the ratio of the time constants, i.e. $[(A_1h_{c1})/(\rho_1V_1c_1)]/[(A_2h_{c2})/(\rho_2V_2c_2)]$ is less than 0.98 or more than 1.02. In an embodiment, this ratio is less than 0.95 or more than 1.05. In an embodiment, the difference $|(A_1h_{c1})/(\rho_1V_1c_1)-(A_2h_{c2})/(\rho_2V_2c_2)|$ is at least $3\times10^{-4}$ s$^{-1}$ or at least $6\times10^{-4}$ s$^{-1}$.

As a result, even if the first and second primary periods of time 411 and 412 have the same starting point, during at least a period of time comprised by both the first primary period of time 411 and the second primary period of time 412, the temperature $T_{s2}(t)$ of the substance or the substances or the other substance or the other substances arranged in the second thermally insulating container 120 is different from the temperature $T_{s1}(t)$ of the substance or the substances arranged in the first thermally insulating container 110. Herein the period of time comprised by the first primary period of time 411 also comprises the time t of the aforementioned temperature profiles $T_{s2}(t)$ and $T_{s1}(t)$. Moreover, at that time, at least one of $T_{s2}(t)$ and $T_{s1}(t)$ is different from the temperature of the environment, $T_o(t)$.

Preferably, the time constants are both determined as discussed above for the case of only one thermally insulating container. More specifically, they both are preferably determined by introducing both the containers 110, 120 into the same second environment 205, and measuring their temperature profiles after the reactions in both the containers have sufficiently slowed down; $T_{s1}(t)$ during the first secondary period of time 421 and $T_{s2}(t)$ during the second secondary period of time 422. Preferably, the secondary periods of time 421, 422 overlap each other by at least 10 % or at least 50 % of the duration of the shorter of the periods, or more preferably they are the same. In case of multiple containers 110, 120, 130, and 140, all the time constants are preferably determined by introducing all the containers 110, 120, 130, 140 into the same second environment 205, and measuring their temperature profiles $T_{si}(t)$, where i=1,2,3, or 4, during the corresponding secondary periods of time (421, 422, 423, 424). From there, the rate of heat release $q_i$ or heat $Q_i=\int q_i$ can be determined as discussed above (see e.g. Eq. 6). Moreover, the total heat capacity needs to be known, received, or measured.

Preferably, all the secondary periods of time 421, 422, 423, 424 at least partially overlap, or more preferably they are the same. In an embodiment, for such a period of time that the first secondary period of time 421 and the second secondary period of time 422 overlap, the first thermally insulating container 110 and the second thermally insulating container 120 are arranged in the same second environment 205. An embodiment comprises determining, for a secondary period of time (421, 422), only one temperature profile $T_o(t)$ for the second environment 205 and measuring, during the secondary period of time (421, 422), at least two temperature profiles $T_{s1}(t)$, $T_{s2}(t)$.

Because of the simplicity, the method is easily parallelized. All the equations 1-6 having a subscript "1" are generally applicable also to any other substance i (e.g. in Figs. 3 and 4 also to i = 2, 3, or 4). Moreover, the skilled person can easily parallelize the method for any finite number of thermally insulating containers (i = 5, 6, 7, ...).

Referring to Figs. 5a-5d, preferably the first secondary period of time 421 starts soon after the latest end point of a primary period of time (411, 412, 413, 414). The first secondary period of time 421 may start at the moment the latest primary period of time (411, 412, 413, 414) ends. In an embodiment, the first secondary period of time 421 starts at most one hour after the end point of a latest primary period of time (411, 412, 413, 414). For example, if the containers 110, 120, 130, 140 are moved to another oven, some time may elapse between the primary and secondary periods of time. However, preferably the time between the primary and secondary periods of time is relatively short to increase the accuracy of measurements and decrease the measurement time.

The values of $\rho_i$, $V_i$ and $c_i$ for all substances $S_i$ can be measured with techniques known to a skilled person. In the alternative, the specific heat capacity and/or the density may be found from literature, and the volume may be measured.

The method has been found particularly suitable for the measurements of reaction heat Q or rate of heat release q of spontaneous reactions of bio-oil. Bio-oil here refers to a substance that

    o is in liquid form at least at the temperature range from 15 °C to 150 °C,
    ∘ comprises some compounds comprising carbon, hydrogen, and oxygen,and
    ∘ has been made in an industrial process from feedstock material of biological origin, such as plants or animals.

The method has been found particularly suitable for the measurements of spontaneous reactions of bio-oil made from plants, such a trees. The method has been found particularly suitable for measuring rate of heat release of spontaneous reactions of pyrolysis oil. In an embodiment, the substance or the substances $S_1$ comprise pyrolysis oil.

When using the method for measuring the heat of a reaction of bio-oil, preferably the temperature $T_o(t)$ of the first environment 200 is from 20 °C to 100 °C for at least a half of the first primary period 411 of time. In addition or alternatively, preferably, the temperature $T_{S1}(t)$ of the substance or substances $S_1$ arranged in the first thermally insulated container 110 is from 20 °C to 100 °C for at least a half of the first primary period 411 of time. Moreover, temperature of the substance is preferably at most 150 °C at all times. These values may apply also for other substances than bio-oils.

After determining heat or rate of heat release produced by a slow reaction of a substance or substances, the kinetic reaction parameters of Eq. 2 can be solved by fitting the measured temperature profiles to the mathematical model described by Eqns. 1-3, and optionally also by Eq. 5. However, the values of the frequency parameter $A_{f1}$ and the energy of the reaction $\Delta H_1$ cannot be independently solved. Instead, values for the activation energy of the reaction $E_{a1}$ and the product $A_{f1}\Delta H_1$ can be obtained. Thus, one can determine at least two kinetic reaction parameters ($E_{a1}$ and $A_{f1}\Delta H_1$) using the heat produced by the slow reaction. By measuring multiple samples, more reaction parameters ($E_{ai}$ and $A_{fi}\Delta H_i$) can be obtained. Statistical techniques can be used to improve the accuracy of the parameters thus detected. As mentioned earlier, these parameters can be seen to describe an average of multiple reactions occurring at the same time in the sample(s).

Moreover, using these two kinetic reaction parameters or a statistical measure (e.g. average) thereof, one can estimate the rate of heat release $q_1$ of the spontaneous reaction. Moreover, a specific rate of heat release, $q_1/(\rho_1 V_1)$ or $q_1/V_1$, can be calculated.

In practice, it may be critical to ensure that material does not heat too much because of the spontaneous reactions. To this end, one has two options:

    (1) Using at least the two kinetic reaction parameters to estimate a storage time such that the remaining reaction heat of the slow reaction is below a critical level. This can be done e.g. in such a way that the remaining reaction heat $\int q_1$, wherein the integral is from an initial storage time to infinity, is so low that, taking into account the total heat capacity $\rho_1 V_1 c_1$, the temperature of the substance will not rise above a critical temperature, even if the container containing the substance was ideally thermally insulated (i.e. adiabatic). In this case, material substantially similar to the substance or substances that have been measured can be stored in a thermally conducting storage for at least the storage time before releasing, shipping, or transferring the material to a customer.

(2) Using at least the two kinetic reaction parameters to estimate a critical heat transfer rate such that the absolute value of the rate of heat release of the slow reaction $|q_1|$ is below the critical heat transfer rate for at least most of the storage time. The critical heat transfer rate may be e.g. substantially equal to the highest absolute value of estimated rate of heat release $q_1$ in the storage conditions. It may, however, be slightly less, since then the excess heat will be stored to the substance itself due to its heat capacity. Moreover, the storage can be equipped with heat transfer equipment (such as a heater and/or a cooler) such that the rate of heat release of the slow reaction can be compensated with the heat transfer equipment. From the storage, the material can be released, shipped, or transferred to a customer.

Some combination of the options (1) and (2) is also possible. E.g. the material may be stored initially in a first storage having a cooler and after some time transferred to a second storage not equipped with a cooler. From the second storage, the material may be delivered to a customer.

As indicated above, Eqns. 2 and 3 may describe multiple reactions on average. Thus, preferably the temperature $T_{s1}(t)$ of the substance during measurements is at least close to the temperature at which the substance is stored before transfer. A computer runs a computer program, which may be sold as a computer program product.

However, the computer or the computer program needs not to

- arrange the substance or the substances in a first thermally insulating container, and
- arrange the thermally insulating container in a first environment, since these steps are performed by an operator.

Moreover, the program may be configured to adjust a temperature inside an oven.

For similar reasons, the computer needs not to arrange a second environment to surround the first thermally insulating container, in particular, when measuring the total heat transfer coefficient. However, when using a temperature controlled oven 210, the computer program may also control the temperature of the oven.

For similar reasons, the computer or the program needs not to

- arrange the substance or the substances or another substance or other substances in at least a second thermally insulating container,
- arrange the second thermally insulating container in the same first environment as the first thermally insulating container,
  in particular, when measuring at least two samples.

For similar reasons, the computer needs not to arrange a second environment to surround both the first thermally insulating container and the second thermally insulated container, in particular, when measuring the total heat transfer coefficients of both the first thermally insulated container and the second thermally insulating container.

Evidently, the computer program is arranged to perform some parts of the method as discussed above. An embodiment of the computer program is, when executed on a computer, arranged to cause the computer further to receive information indicative of a total heat capacity $\rho_1 V_1 c_1$ of the substance or the substances $S_1$ arranged in the first thermally insulating container 110. An embodiment of the computer program is, when executed on a computer, arranged to cause the computer further to receive information indicative of a total heat capacity $\rho_2 V_2 c_2$ of the substance or the substances or the other substance or the other substances $S_2$ arranged in the second thermally insulating container 120.

The computer program is arranged to receive the measured temperature profiles of the first sample $S_1$. Based on the received temperature profiles (and the received total heat capacity of the sample $S_1$), the program is configured to determine the heat $Q_1$ or the rate of heat release $q_1$ of the reaction of the substance or substances $S_1$ arranged in the first thermally insulating container 110. The program may be configured to receive the temperature profiles of the other samples $S_2$ and the total heat capacity of the other sample $S_2$ and to further determine the heat $Q_2$ or the rate of heat release $q_2$ of the reaction of the substance or substances $S_2$ arranged in the second thermally insulating container 120.

An arrangement may comprise the devices described above. However, the equipment needs not to "arrange" the things discussed above in connection with the computer program, since these steps are performed by an operator of the arrangement. For example, the control unit 300 may comprise a computer, which is arranged to run the aforementioned computer program.

**Claims**

1. A method for determining heat ($Q_1$) or rate of heat release ($q_1$) of a slow reaction of a substance or substances ($S_1$), wherein the rate of heat release or consumption of the slow reaction is less than 0.5 W/kg, the method comprising

- arranging the substance or the substances ($S_1$) in a first thermally insulating container (110),
- measuring a temporal temperature profile ($T_{s1}(t)$) of the substance or the substances ($S_1$) arranged in the first thermally insulating container (110) during a first primary period of time (411) and as function of time ($t$),
- determining, during the first primary period of time (411) and as function of time ($t$), a temporal temperature profile ($T_o(t)$) of the environment (200) in which the first thermally insulating container (110) is arranged during the first primary period of time (411),
- measuring a temporal temperature profile ($T_{s1}(t)$) of the substance or the substances ($S_1$) arranged in the first thermally insulating container (110) during a first secondary period of time (421) and as function of time ($t$),
- determining, during the first secondary period of time (421) and as function of time ($t$), a temporal temperature profile ($T_o(t)$) of the environment (205) in which the first thermally insulating container (110) is arranged during the first secondary period of time (421), and
- determining the heat ($Q_1$) or the rate of heat release ($q_1$) of the reaction of the substance or substances ($S_1$) arranged in the first thermally insulating container (110) using

   ◦ the temporal temperature profiles ($T_{s1}(t)$) of the substance or the substances ($S_1$) arranged in the first thermally insulating container (110) during the first primary period of time (411) and the first secondary period of time (421),
   ◦ the temporal temperature profile ($T_o(t)$) during the first primary period of time (411) of the environment (200) in which the first thermally insulating container (110) is arranged during the first primary period of time (411), and the temporal temperature profile ($T_o(t)$) during the first secondary period of time (421) of the environment (205) in which the first thermally insulating container (110) is arranged during the first secondary period of time (421); and
   ◦ information indicative of the total heat capacity $\rho_1 V_1 c_1$ of the substance or the substances ($S_1$) arranged in the first thermally insulating container (110), wherein $\rho_1$ is the density of the substance or the substances, $V_1$ is the volume of the substance or the substances, and $c_1$ is the heat capacity of the substance or the substances, wherein

- for at least a part of the first primary period of time (411), the temperature ($T_{s1}(t)$) of the substance or the substances ($S_1$) arranged in the first thermally insulating container (110) is different from the temperature ($T_o(t)$) of the environment (200) in which the first thermally insulating container (110) is arranged at that time, and
- the average temperature of the environment (200) in which the first thermally insulating container (110) is arranged during the first primary period of time is (411) different from the average temperature of the environment (205) in which the first thermally insulating container (110) is arranged during the first secondary period of time (421).

2. The method of claim 1, wherein

- the first secondary period of time (421) starts after such an instance of time, that

   ◦ the absolute temperature difference between the substance ($S_1$) and the environment (200), $|T_{s1}(t)-T_o(t)|$, has decreased to at most 20 % of the maximum absolute temperature difference between the substance ($S_1$) and the environment (200), $\max(|T_{s1}(t)-T_o(t)|)$, and/or
   ◦ the first primary period of time (411) has lasted at least five times the time ($t\Delta T1,max$) when the maximum absolute temperature difference occurred as measured from the start of the first primary period of time (411), and

- the medium surrounding the first thermally insulating container (110) is in the same state during the first primary period of time (411) and the first secondary period of time (421).

3. The method of claim 1 or 2 comprising

- arranging the substance or the substances or some other substance or some other substances ($S_2$) in at least a second thermally insulating container (120),

- arranging, during a second primary period of time (412), the second thermally insulating container (120) in the same environment (200) in which the first thermally insulating container (110) is arranged for at least a part of that period of time (412),
- measuring a temporal temperature profile ($T_{s2}(t)$) of the substance or the substances or the other substance or the other substances ($S_2$) arranged in the second thermally insulating container (120) during the second primary period of time (412) and as function of time (t),
- determining, during the second primary period of time (412) and as function of time (t), a temporal temperature profile ($T_0(t)$) of the environment (200) in which the second thermally insulating container (120) is arranged during the second primary period of time (412),
- arranging, during a second secondary period of time (422), the second thermally insulating container (120) in the same environment (205) in which the first thermally insulating container (110) is arranged for at least a part of that period of time (422),
- measuring a temporal temperature profile ($T_{s2}(t)$) of the substance or the substances or the other substance or the other substances ($S_2$) arranged in the second thermally insulating container (120) during the second secondary period of time (422) and as function of time (t),
- determining, during the second secondary period of time (422) and as function of time (t), a temporal temperature profile ($T_0(t)$) of the environment (205) in which the second thermally insulating container (120) is arranged during the second secondary period of time (422), and
- determining the heat or the rate of heat release of the reaction of the substance or substances or the other substance or the other substances ($S_2$) arranged in the second thermally insulating container (120) using

  ◦ the temporal temperature profiles ($T_{s2}(t)$) of the substance or the substances or the other substance or the other substances ($S_2$) arranged in the second thermally insulating container (120) during the second primary period of time (412) and the second secondary period of time (422),
  ◦ the temporal temperature profile ($T_0(t)$) during the second primary period of time (412) of the environment (200) in which the second thermally insulating container (110) is arranged during the second primary period of time (412), and the temporal temperature profile ($T_0(t)$) during the second secondary period of time (422) of the environment (205) in which the second thermally insulating container (120) is arranged during the second secondary period of time (422), and
  ◦ information indicative of the total heat capacity of the substance or the substances or the other substance or the other substances ($S_2$) arranged in the second thermally insulating container (120).

4. The method of claim 3, wherein

   - the first primary period of time (411) at least partly overlaps the second primary period of time (412) for example by at least 10 % of the duration of the shorter of the periods, and
   - for the period of time that the first primary period of time (411) and the second primary period of time (412) overlap, the first thermally insulating container (110) and the second thermally insulating container (120) are arranged in the same environment (200);
   preferably
   - the first primary period of time (411) is the same as the second primary period of time (412).

5. The method of claim 3 or 4, wherein

   - the first secondary period of time (421) at least partly overlaps the second secondary period of time (422) for example by at least 10 % of the duration of the shorter of the periods, and
   - for the period of time that the first secondary period of time (421) and the second secondary period of time (422) overlap, the first thermally insulating container (110) and the second thermally insulating container (120) are arranged in the same environment (205);
   preferably
   - the first secondary period of time (421) is the same as the second secondary period of time (422).

6. The method of any of the claims 1 to 5, wherein

   - the reactions of the substance or the substances ($S_1$) in the first thermally insulating container (110) are so slow, that at all times during the first primary period of time (421), the specific absolute value of the rate of heat release $|q_1(t)/(\rho_1 V_1)|$ is less than 2 W/kg, and/or the absolute value of the rate of adiabatic temperature change

$|q_1(t)/(\rho_1 V_1 c_1)|$ is less than 2 °C/h and/or
- the first primary period of time (411) spans at least 50 hours.

7. The method of any of the claims 1 to 6, wherein

- the time average over the first secondary period of time (421) of the temporal temperature profile ($T_0(t)$) of the environment (205), in which the first thermally insulating container (110) is arranged during the first secondary period of time (421) is less than the time average over the first primary period of time (411) of the temporal temperature profile ($T_0(t)$) of the environment (200), in which the first thermally insulating container (110) is arranged during the first primary period of time (411); optionally by at least 1 °C; and optionally by at most 50 °C.

8. The method of any of the claims 1 to 7, wherein for at least a half of the first primary period of time (411),

- the temperature of the environment ($T_0(t)$), in which the first thermally insulating container (110) is arranged during the first primary period of time (411), is from 20 °C to 100 °C and/or
- the temperature ($T_{s1}(t)$) of the substance or substances ($S_1$) arranged in the first thermally insulated container (110), during the first primary period of time (411), is from 20 °C to 150 °C.

9. The method of any of the claims 1 to 8, wherein

- the first thermally insulated container (110) is arranged in a same temperature-controlled environment (200, 205) of a device (210) surrounding the temperature-controlled environment during

  ∘ the first primary period of time (411) and
  ∘ the first secondary period of time (421).

10. The method of any of the claims 1 to 9, wherein

- the substance or the substances ($S_1$) comprise bio-oil, such as bio-oil that can be manufactured from feedstock material of biological origin in an industrial process;
preferably
- the substance or the substances ($S_1$) comprise pyrolysis oil.

11. The method of any of the claims 1 to 10, wherein at all times during the first primary period of time (411),

- the temperature ($T_0(t)$) of the first environment and the temperature ($T_{s1}(t)$) of the substance or substances ($S_1$) are less than 250 °C, and
- the absolute temperature difference between the substance ($S_1$) and the first environment (200), i.e. $|T_{s1}(t)-T_0(t)|$, is less than 150 °C, whereby
- heat transfer, including radiative heat transfer, between the substance ($S_1$) in the first thermally insulated container (110) and the environment (200) can be assumed to be proportional to the temperature difference ($T_{s1}(t)-T_0(t)$) between the first thermally insulated container (110) and the environment (200).

12. The method of any of the claims 1 to 11, wherein

- at least 90 wt% of the substance or the substances ($S_1$) is in liquid form, and
- the first thermally insulating container (110) is selected in such a way that that the heat capacity of the first thermally insulating container (110) is at most 15 % of the total heat capacity ($\rho_1 V_1 c_1$) of the substance or the substances ($S_1$) arranged in the first thermally insulating container (110).

13. The method of any of the claims 1 to 12, comprising

- determining at least two kinetic reaction parameters using the heat ($Q_1$) or rate of heat release ($q_1$) produced by the slow reaction,
- estimating, using the kinetic reaction parameters, at least one of

  ∘ a storage time such that the remaining reaction heat of the slow reaction is below a critical level and
  ∘ a critical heat transfer rate such that the absolute value of the rate of heat release

of the slow reaction is below the critical heat transfer rate for at least 75 % of a storage time,

- storing material similar to the substance or substances ($S_1$)

    ◦ for at least the storage time and/or
    ◦ in a container equipped with heat transfer equipment having the capability of compensating a rate of heat release of which absolute value is at most the critical heat transfer rate; and

- thereafter, releasing, shipping, or transferring the material similar to the substance or substances ($S_1$) to a customer.

14. A computer program that, when executed on a computer, causes the computer to

- receive information indicative of a temporal temperature profile ($T_{s1}(t)$) of a substance or substances ($S_1$) arranged in a first thermally insulating container (110) for a first primary period of time (411) and as function of time (t),
- receive information indicative of a temporal temperature profile ($T_0(t)$), for the first primary period of time (411) and as function of time (t), of the environment (200) in which the first thermally insulating container (110) is arranged during the first primary period of time (411),
- receive information indicative of a temporal temperature profile ($T_{s1}(t)$) of the substance or the substances ($S_1$) arranged in the first thermally insulating container (110) for a first secondary period of time (421) and as function of time (t),
- receive information indicative of a temporal temperature profile ($T_0(t)$), for the first secondary period of time (421) and as function of time (t), of the environment (205) in which the first thermally insulating container (110) is arranged for the first secondary period of time (421), and
- determine the heat ($Q_1$) or the rate of heat release ($q_1$) of the reaction of the substance or substances ($S_1$) arranged in the first thermally insulating container (110) using

    ◦ the temporal temperature profiles ($T_{s1}(t)$) of the substance or the substances ($S_1$) arranged in the first thermally insulating container (110) for the first primary period of time (411) and for the first secondary period of time (421),
    ◦ the temporal temperature profile ($T_0(t)$), for the first primary period of time (411), of the environment (200) in which the first thermally insulating container (110) is arranged during the first primary period of time (411) and the temporal temperature profile ($T_0(t)$), for the first secondary period of time (421), of the environment (205) in which the first thermally insulating container (110) is arranged during the first secondary period of time (421), and
    ◦ information indicative of the total heat capacity $\rho_1 V_1 c_1$ of the substance or the substances ($S_1$) arranged in the first thermally insulating container (110), wherein $\rho_1$ is the density of the substance or the substances, $V_1$ is the volume of the substance or the substances, and $c_1$ is the heat capacity of the substance or the substances, wherein

- for at least a part of the first primary period of time (411), the temperature ($T_{s1}(t)$) of the substance or the substances ($S_1$) arranged in the first thermally insulating container (110) is different from the temperature ($T_0(t)$) of the environment (200) in which the first thermally insulating container (110) is arranged at that time, and
- the average temperature of the environment (200) in which the first thermally insulating container (110) is arranged during the first primary period of time (411) is different from the average temperature of the environment (205) in which the first thermally insulating container (110) is arranged during the first secondary period of time (421).

15. The computer program of claim 14 that, when executed on a computer, causes the computer further to

- receive information indicative of a temporal temperature profile ($T_{s2}(t)$) of the substance or the substances or another substance or other substances ($S_2$) arranged in a second thermally insulating container (120) for a second primary period of time (412) and as function of time (t),
- receive information indicative of a temporal temperature profile ($T_0(t)$), for the second primary period of time (412) and as function of time (t), of the environment (200) in which the second thermally insulating container (120) is arranged during the second primary period of time (412),
- receive information indicative of a temporal temperature profile ($T_{s2}(t)$) of the substance or the substances or the other substance or the other substances ($S_2$) arranged in the second thermally insulating container (120) for a second secondary period of time (422) and as function of time (t),

- receive information indicative of a temporal temperature profile ($T_0(t)$), for the second secondary period of time (422) and as function of time (t), of the environment (205) in which the second thermally insulating container (120) is arranged for the second secondary period of time (422), and
- determine the heat ($Q_2$) or the rate of heat release ($q_2$) of the reaction of the substance or the substances or the other substance or the other substances ($S_2$) arranged in the second thermally insulating container (120) using

    ∘ the temporal temperature profiles ($T_{s2}(t)$) of the substance or the substances or the other substance or the other substances ($S_2$) arranged in the second thermally insulating container (120) for the second primary period of time (412) and for the second secondary period of time (422),
    ∘ the temporal temperature profile ($T_0(t)$), for the second primary period of time (412), of the environment (200) in which the second thermally insulating container (120) is arranged during the second primary period of time (412) and the temporal temperature profile ($T_0(t)$), for the second secondary period of time (422), of the environment (205) in which the second thermally insulating container (120) is arranged during the second secondary period of time (422), and
    ∘ information indicative of the total heat capacity of the substance or the substances or the other substance or the other substances ($S_2$) arranged in the second thermally insulating container (120).

**Patentansprüche**

1. Verfahren zur Ermittlung der Wärme ($Q_1$) oder Rate der Wärmefreisetzung ($q_1$) einer langsamen Reaktion einer Substanz oder von Substanzen ($S_1$), wobei die Rate der Wärmefreisetzung oder des Wärmeverbrauchs der langsamen Reaktion weniger als 0,5 W/kg beträgt, wobei das Verfahren Folgendes umfasst:

- Anordnen der Substanz oder der Substanzen ($S_1$) in einem ersten wärmeisolierenden Behälter (110),
- Messen eines zeitlichen Temperaturprofils ($T_{s1}(t)$) der Substanz oder der Substanzen ($S_1$), die im ersten wärmeisolierenden Behälter (110) angeordnet ist/sind, während eines ersten primären Zeitraums (411) und in Abhängigkeit von der Zeit (t),
- Ermitteln, während des ersten primären Zeitraums (411) und in Abhängigkeit von der Zeit (t), eines zeitlichen Temperaturprofils ($T_0(t)$) der Umgebung (200), in welcher der erste wärmeisolierende Behälter (110) während des ersten primären Zeitraums (411) angeordnet ist,
- Messen eines zeitlichen Temperaturprofils ($T_{s1}(t)$) der Substanz oder der Substanzen ($S_1$), die im ersten wärmeisolierenden Behälter (110) angeordnet ist/sind, während eines ersten sekundären Zeitraums (421) und in Abhängigkeit von der Zeit (t),
- Ermitteln, während des ersten sekundären Zeitraums (421) und in Abhängigkeit von der Zeit (t), eines zeitlichen Temperaturprofils ($T_0(t)$) der Umgebung (205), in welcher der erste wärmeisolierende Behälter (110) während des ersten sekundären Zeitraums (421) angeordnet ist, und
- Ermitteln der Wärme ($Q_1$) oder Rate der Wärmefreisetzung ($q_1$) der Reaktion der Substanz oder der Substanzen ($S_1$), die im ersten wärmeisolierenden Behälter (110) angeordnet ist/sind, unter Verwendung

    ∘ der zeitlichen Temperaturprofile ($T_{s1}(t)$) der Substanz oder der Substanzen ($S_1$), die im ersten wärmeisolierenden Behälter (110) während des ersten primären Zeitraums (411) und des ersten sekundären Zeitraums (421) angeordnet ist/sind,
    ∘ des zeitlichen Temperaturprofils ($T_0(t)$), während des ersten primären Zeitraums (411), der Umgebung (200), in welcher der erste wärmeisolierende Behälter (110) während des ersten primären Zeitraums (411) angeordnet ist, und des zeitlichen Temperaturprofils ($T_0(t)$), während des ersten sekundären Zeitraums (421), der Umgebung (205), in welcher der erste wärmeisolierende Behälter (110) während des ersten sekundären Zeitraums (421) angeordnet ist; und
    ∘ einer Information, die die gesamte Wärmekapazität $\rho_1 V_1 c_1$ der Substanz oder der Substanzen ($S_1$), die im ersten wärmeisolierenden Behälter (110) angeordnet ist/sind, angibt, wobei $\rho_1$ für die Dichte der Substanz oder der Substanzen steht, $V_1$ für das Volumen der Substanz oder der Substanzen steht und $c_1$ für die Wärmekapazität der Substanz oder der Substanzen steht, wobei

- für zumindest einen Teil des ersten primären Zeitraums (411) die Temperatur ($T_{s1}(t)$) der Substanz oder der Substanzen ($S_1$), die im ersten wärmeisolierenden Behälter (110) angeordnet ist/sind, verschieden ist von der

Temperatur ($T_o$(t)) der Umgebung (200), in welcher der erste wärmeisolierende Behälter (110) in dieser Zeit angeordnet ist, und

- die mittlere Temperatur der Umgebung (200), in welcher der erste wärmeisolierende Behälter (110) während des ersten primären Zeitraums (411) angeordnet ist, verschieden ist von der mittleren Temperatur der Umgebung (205), in welcher der erste wärmeisolierende Behälter (110) während des ersten sekundären Zeitraums (421) angeordnet ist.

2. Verfahren nach Anspruch 1, wobei

- der erste sekundäre Zeitraum (421) nach einem derartigen Zeitpunkt beginnt, bei dem

  ◦ die absolute Temperaturdifferenz zwischen der Substanz ($S_1$) und der Umgebung (200), $|T_{s1}(t)-T_o(t)|$, sich auf höchstens 20 % der maximalen absoluten Temperaturdifferenz zwischen der Substanz ($S_1$) und der Umgebung (200), $\max(|T_{s1}(t)-T_o(t)|)$, verringert hat und/oder
  ◦ der erste primäre Zeitraum (411) mindestens das Fünffache der Zeit (t△T1,max) andauerte, als die maximale absolute Temperaturdifferenz bei Messung ab dem Beginn des ersten primären Zeitraums (411) auftrat, und

- das Medium, das den ersten wärmeisolierenden Behälter (110) umgibt, während des ersten primären Zeitraums (411) und des ersten sekundären Zeitraums (421) in demselben Zustand ist.

3. Verfahren nach Anspruch 1 oder 2, umfassend:

- Anordnen der Substanz oder der Substanzen oder irgendeiner anderen Substanz oder irgendwelcher anderer Substanzen ($S_2$) in mindestens einem zweiten wärmeisolierenden Behälter (120),
- Anordnen, während eines zweiten primären Zeitraums (412), des zweiten wärmeisolierenden Behälters (120) in derselben Umgebung (200), in welcher der erste wärmeisolierende Behälter (110) für zumindest einen Teil dieses Zeitraums (412) angeordnet ist,
- Messen eines zeitlichen Temperaturprofils ($T_{s2}$(t)) der Substanz oder der Substanzen oder der anderen Substanz oder der anderen Substanzen ($S_2$), die im zweiten wärmeisolierenden Behälter (120) angeordnet ist/sind, während des zweiten primären Zeitraums (412) und in Abhängigkeit von der Zeit (t),
- Ermitteln, während des zweiten primären Zeitraums (412) und in Abhängigkeit von der Zeit (t), eines zeitlichen Temperaturprofils ($T_o$(t)) der Umgebung (200), in welcher der zweite wärmeisolierende Behälter (120) während des zweiten primären Zeitraums (412) angeordnet ist,
- Anordnen, während eines zweiten sekundären Zeitraums (422), des zweiten wärmeisolierenden Behälters (120) in derselben Umgebung (205), in welcher der erste wärmeisolierende Behälter (110) für zumindest einen Teil dieses Zeitraums (422) angeordnet ist,
- Messen eines zeitlichen Temperaturprofils ($T_{s2}$(t)) der Substanz oder der Substanzen oder der anderen Substanz oder der anderen Substanzen ($S_2$), die im zweiten wärmeisolierenden Behälter (120) angeordnet ist/sind, während des zweiten sekundären Zeitraums (422) und in Abhängigkeit von der Zeit (t),
- Ermitteln, während des zweiten sekundären Zeitraums (422) und in Abhängigkeit von der Zeit (t), eines zeitlichen Temperaturprofils ($T_o$(t)) der Umgebung (205), in welcher der zweite wärmeisolierende Behälter (120) während des zweiten sekundären Zeitraums (422) angeordnet ist, und
- Ermitteln der Wärme oder Rate der Wärmefreisetzung der Reaktion der Substanz oder der Substanzen oder der anderen Substanz oder der anderen Substanzen ($S_2$), die im zweiten wärmeisolierenden Behälter (120) angeordnet ist/sind, unter Verwendung

  ◦ der zeitlichen Temperaturprofile ($T_{s2}$(t)) der Substanz oder der Substanzen oder der anderen Substanz oder der anderen Substanzen ($S_2$), die im zweiten wärmeisolierenden Behälter (120) während des zweiten primären Zeitraums (412) und des zweiten sekundären Zeitraums (422) angeordnet ist/sind,
  ◦ des zeitlichen Temperaturprofils ($T_o$(t)), während des zweiten primären Zeitraums (412), der Umgebung (200), in welcher der zweite wärmeisolierende Behälter (110) während des zweiten primären Zeitraums (412) angeordnet ist, und des zeitlichen Temperaturprofils ($T_o$(t)), während des zweiten sekundären Zeitraums (422), der Umgebung (205), in welcher der zweite wärmeisolierende Behälter (120) während des zweiten sekundären Zeitraums (422) angeordnet ist, und
  ◦ einer Information, die die gesamte Wärmekapazität der Substanz oder der Substanzen oder der anderen Substanz oder der anderen Substanzen ($S_2$), die im zweiten wärmeisolierenden Behälter (120) angeordnet ist/sind, angibt.

4. Verfahren nach Anspruch 3, wobei

   - der erste primäre Zeitraum (411) zumindest teilweise den zweiten primären Zeitraum (412) beispielsweise um mindestens 10 % der Dauer des kürzeren der Zeiträume überlappt und
   - für den Zeitraum, den sich der erste primäre Zeitraum (411) und der zweite primäre Zeitraum (412) überlappen, der erste wärmeisolierende Behälter (110) und der zweite wärmeisolierende Behälter (120) in derselben Umgebung (200) angeordnet sind;
   vorzugsweise
   - der erste primäre Zeitraum (411) der gleiche wie der zweite primäre Zeitraum (412) ist.

5. Verfahren nach Anspruch 3 oder 4, wobei

   - der erste sekundäre Zeitraum (421) zumindest teilweise den zweiten sekundären Zeitraum (422) beispielsweise um mindestens 10 % der Dauer des kürzeren der Zeiträume überlappt und
   - für den Zeitraum, den sich der erste sekundäre Zeitraum (421) und der zweite sekundäre Zeitraum (422) überlappen, der erste wärmeisolierende Behälter (110) und der zweite wärmeisolierende Behälter (120) in derselben Umgebung (205) angeordnet sind; vorzugsweise
   - der erste sekundäre Zeitraum (421) der gleiche wie der zweite sekundäre Zeitraum (422) ist.

6. Verfahren nach irgendeinem der Ansprüche 1 bis 5, wobei

   - die Reaktionen der Substanz oder der Substanzen $(S_1)$ im ersten wärmeisolierenden Behälter (110) so langsam sind, dass jederzeit während des ersten primären Zeitraums (421) der spezifische Absolutwert der Rate der Wärmefreisetzung $|q_1(t)/(\rho_1 V_1)|$ kleiner als 2 W/kg ist und/oder der Absolutwert der Rate der adiabatischen Temperaturänderung $|q_1(t)/(\rho_1 V_1 c_1)|$ kleiner als 2 °C/h ist und/oder
   - der erste primäre Zeitraum (411) sich über mindestens 50 Stunden erstreckt.

7. Verfahren nach irgendeinem der Ansprüche 1 bis 6, wobei

   - der zeitliche Mittelwert über den ersten sekundären Zeitraum (421) des zeitlichen Temperaturprofils $(T_o(t))$ der Umgebung (205), in welcher der erste wärmeisolierende Behälter (110) während des ersten sekundären Zeitraums (421) angeordnet ist, kleiner ist als der zeitliche Mittelwert über den ersten primären Zeitraum (411) des zeitlichen Temperaturprofils $(T_o(t))$ der Umgebung (200), in welcher der erste wärmeisolierende Behälter (110) während des ersten primären Zeitraums (411) angeordnet ist; gegebenenfalls um mindestens 1 °C; und gegebenenfalls um höchstens 50 °C.

8. Verfahren nach irgendeinem der Ansprüche 1 bis 7, wobei für mindestens eine Hälfte des ersten primären Zeitraums (411)

   - die Temperatur $(T_o(t))$ der Umgebung, in welcher der erste wärmeisolierende Behälter (110) während des ersten primären Zeitraums (411) angeordnet ist, 20 °C bis 100 °C beträgt und/oder
   - die Temperatur $(T_{s1}(t))$ der Substanz oder der Substanzen $(S_1)$, die im ersten wärmeisolierenden Behälter (110) während des ersten primären Zeitraums (411) angeordnet ist/sind, 20 °C bis 150 °C beträgt.

9. Verfahren nach irgendeinem der Ansprüche 1 bis 8, wobei

   - der erste wärmeisolierende Behälter (110) in einer gleichen temperaturgeregelten Umgebung (200, 205) einer Vorrichtung (210) angeordnet ist, die die temperaturgeregelte Umgebung während

     ∘ des ersten primären Zeitraums (411) und
     ∘ des ersten sekundären Zeitraums (421) umgibt.

10. Verfahren nach irgendeinem der Ansprüche 1 bis 9, wobei

    - die Substanz oder die Substanzen $(S_1)$ ein Bio-Öl wie beispielsweise ein Bio-Öl, das aus Einsatzmaterial biologischen Ursprungs in einem industriellen Prozess hergestellt werden kann, umfasst/umfassen; vorzugsweise
    - die Substanz oder die Substanzen $(S_1)$ Pyrolyseöl umfasst/umfassen.

**11.** Verfahren nach irgendeinem der Ansprüche 1 bis 10, wobei jederzeit während des ersten primären Zeitraums (411)

- die Temperatur ($T_o(t)$) der ersten Umgebung und die Temperatur ($T_{s1}(t)$) der Substanz oder der Substanzen ($S_1$) weniger als 250 °C beträgt und
- die absolute Temperaturdifferenz zwischen der Substanz ($S_1$) und der ersten Umgebung (200), d. h. $|T_{s1}(t)-T_o(t)|$, kleiner als 150 °C ist, wodurch
- eine Wärmeübertragung, einschließlich einer Wärmeübertragung durch Strahlung, zwischen der Substanz ($S_1$) im ersten wärmeisolierenden Behälter (110) und der Umgebung (200) als proportional der Temperaturdifferenz ($T_{s1}(t)-T_o(t)$) zwischen dem ersten wärmeisolierenden Behälter (110) und der Umgebung (200) angenommen werden kann.

**12.** Verfahren nach irgendeinem der Ansprüche 1 bis 11, wobei

- mindestens 90 Gew.-% der Substanz oder der Substanzen ($S_1$) in flüssiger Form vorliegen und
- der erste wärmeisolierende Behälter (110) derart ausgewählt ist, dass die Wärmekapazität des ersten wärmeisolierenden Behälters (110) höchstens 15 % der gesamten Wärmekapazität ($\rho_1 V_1 c_1$) der Substanz oder der Substanzen ($S_1$), die im ersten wärmeisolierenden Behälter (110) angeordnet ist/sind, ausmacht.

**13.** Verfahren nach irgendeinem der Ansprüche 1 bis 12, umfassend

- Ermitteln von mindestens zwei kinetischen Reaktionsparametern unter Verwendung der durch die langsame Reaktion erzeugten Wärme ($Q_1$) oder Rate der Wärmefreisetzung ($q_1$),
- unter Verwendung der kinetischen Reaktionsparameter Einschätzen von mindestens einem von:

  ◦ einer Lagerungszeit derart, dass die verbleibende Reaktionswärme der langsamen Reaktion unter einem kritischen Niveau liegt und
  ◦ einer kritischen Wärmeübertragungsrate derart, dass der Absolutwert der Rate der Wärmefreisetzung der langsamen Reaktion für mindestens 75 % der Lagerungszeit unter der kritischen Wärmeübertragungsrate liegt,

- Lagern von Material, das der Substanz oder den Substanzen ($S_1$) ähnlich ist,

  ◦ für mindestens die Lagerungszeit und/oder
  ◦ in einem Behälter, der mit einer Wärmeübertragungseinrichtung versehen ist, die die Fähigkeit zur Kompensierung einer Rate der Wärmefreisetzung aufweist, deren Absolutwert höchstens die kritische Wärmeübertragungsrate ist; und

- danach Aushändigen, Versenden oder Übergeben des Materials, das der Substanz oder den Substanzen ($S_1$) ähnlich ist, an einen Kunden.

**14.** Computerprogramm, das, wenn es auf einem Computer ausgeführt wird, den Computer dazu veranlasst,

- eine Information zu empfangen, die ein zeitliches Temperaturprofil ($T_{s1}(t)$) einer Substanz oder von Substanzen ($S_1$), die in einem ersten wärmeisolierenden Behälter (110) angeordnet ist/sind, für einen ersten primären Zeitraum (411) und in Abhängigkeit von der Zeit ($t$) angibt,
- eine Information zu empfangen, die ein zeitliches Temperaturprofil ($T_o(t)$), für den ersten primären Zeitraum (411) und in Abhängigkeit von der Zeit ($t$), der Umgebung (200), in welcher der erste wärmeisolierende Behälter (110) während des ersten primären Zeitraums (411) angeordnet ist, angibt,
- eine Information zu empfangen, die ein zeitliches Temperaturprofil ($T_{s1}(t)$) der Substanz oder der Substanzen ($S_1$), die im ersten wärmeisolierenden Behälter (110) angeordnet ist/sind, für einen ersten sekundären Zeitraum (421) und in Abhängigkeit von der Zeit ($t$) angibt,
- eine Information zu empfangen, die ein zeitliches Temperaturprofil ($T_o(t)$), für den ersten sekundären Zeitraum (421) und in Abhängigkeit von der Zeit ($t$), der Umgebung (205), in welcher der erste wärmeisolierende Behälter (110) für den ersten sekundären Zeitraum (421) angeordnet ist, angibt, und
- die Wärme ($Q_1$) oder Rate der Wärmefreisetzung ($q_1$) der Reaktion der Substanz oder der Substanzen ($S_1$), die im ersten wärmeisolierenden Behälter (110) angeordnet ist/sind, zu ermitteln unter Verwendung

  ◦ der zeitlichen Temperaturprofile ($T_{s1}(t)$) der Substanz oder der Substanzen ($S_1$), die im ersten wärmei-

solierenden Behälter (110) für den ersten primären Zeitraum (411) und für den ersten sekundären Zeitraum (421) angeordnet ist/sind,

◦ des zeitlichen Temperaturprofils ($T_0$(t)), für den ersten primären Zeitraum (411), der Umgebung (200), in welcher der erste wärmeisolierende Behälter (110) während des ersten primären Zeitraums (411) angeordnet ist, und des zeitlichen Temperaturprofils ($T_0$(t)), für den ersten sekundären Zeitraum (421), der Umgebung (205), in welcher der erste wärmeisolierende Behälter (110) während des ersten sekundären Zeitraums (421) angeordnet ist, und

◦ einer Information, die die gesamte Wärmekapazität $\rho_1 V_1 c_1$ der Substanz oder der Substanzen ($S_1$), die im ersten wärmeisolierenden Behälter (110) angeordnet ist/sind, angibt, wobei $\rho_1$ für die Dichte der Substanz oder der Substanzen steht, $V_1$ für das Volumen der Substanz oder der Substanzen steht und $c_1$ für die Wärmekapazität der Substanz oder der Substanzen steht, wobei

- für zumindest einen Teil des ersten primären Zeitraums (411) die Temperatur ($T_{s1}$(t)) der Substanz oder der Substanzen ($S_1$), die im ersten wärmeisolierenden Behälter (110) angeordnet ist/sind, verschieden ist von der Temperatur ($T_0$(t)) der Umgebung (200), in welcher der erste wärmeisolierende Behälter (110) in dieser Zeit angeordnet ist, und

- die mittlere Temperatur der Umgebung (200), in welcher der erste wärmeisolierende Behälter (110) während des ersten primären Zeitraums (411) angeordnet ist, verschieden ist von der mittleren Temperatur der Umgebung (205), in welcher der erste wärmeisolierende Behälter (110) während des ersten sekundären Zeitraums (421) angeordnet ist.

**15.** Computerprogramm nach Anspruch 14, das, wenn es auf einem Computer ausgeführt wird, den Computer dazu veranlasst, ferner

- eine Information zu empfangen, die ein zeitliches Temperaturprofil ($T_{s2}$(t)) der Substanz oder der Substanzen oder einer anderen Substanz oder anderer Substanzen ($S_2$), die in einem zweiten wärmeisolierenden Behälter (120) angeordnet ist/sind, für einen zweiten primären Zeitraum (412) und in Abhängigkeit von der Zeit (t) angibt,

- eine Information zu empfangen, die ein zeitliches Temperaturprofil ($T_0$(t)), für den zweiten primären Zeitraum (412) und in Abhängigkeit von der Zeit (t), der Umgebung (200), in welcher der zweite wärmeisolierende Behälter (120) während des zweiten primären Zeitraums (412) angeordnet ist, angibt,

- eine Information zu empfangen, die ein zeitliches Temperaturprofil ($T_{s2}$(t)) der Substanz oder der Substanzen oder der anderen Substanz oder der anderen Substanzen ($S_2$), die im zweiten wärmeisolierenden Behälter (120) angeordnet ist/sind, für einen zweiten sekundären Zeitraum (422) und in Abhängigkeit von der Zeit (t) angibt,

- eine Information zu empfangen, die ein zeitliches Temperaturprofil ($T_0$(t)), für den zweiten sekundären Zeitraum (422) und in Abhängigkeit von der Zeit (t), der Umgebung (205), in welcher der zweite wärmeisolierende Behälter (120) für den zweiten sekundären Zeitraum (422) angeordnet ist, angibt, und

- die Wärme ($Q_2$) oder Rate der Wärmefreisetzung ($q_2$) der Reaktion der Substanz oder der Substanzen oder der anderen Substanz oder der anderen Substanzen ($S_2$), die im zweiten wärmeisolierenden Behälter (120) angeordnet ist/sind, zu ermitteln unter Verwendung

◦ der zeitlichen Temperaturprofile ($T_{s2}$(t)) der Substanz oder der Substanzen oder der anderen Substanz oder der anderen Substanzen ($S_2$), die im zweiten wärmeisolierenden Behälter (120) für den zweiten primären Zeitraum (412) und für den zweiten sekundären Zeitraum (422) angeordnet ist/sind,

◦ des zeitlichen Temperaturprofils ($T_0$(t)), für den zweiten primären Zeitraum (412), der Umgebung (200), in welcher der zweite wärmeisolierende Behälter (120) während des zweiten primären Zeitraums (412) angeordnet ist, und des zeitlichen Temperaturprofils ($T_0$(t)), für den zweiten sekundären Zeitraum (422), der Umgebung (205), in welcher der zweite wärmeisolierende Behälter (120) während des zweiten sekundären Zeitraums (422) angeordnet ist, und

◦ einer Information, die die gesamte Wärmekapazität der Substanz oder der Substanzen oder der anderen Substanz oder der anderen Substanzen ($S_2$), die im zweiten wärmeisolierenden Behälter (120) angeordnet ist/sind, angibt.

**Revendications**

**1.** Procédé pour déterminer la chaleur ($Q_1$) ou le taux de dégagement de chaleur ($q_1$) d'une réaction lente d'une substance ou de substances ($S_1$), dans lequel le taux de dégagement de chaleur ou la consommation de la réaction lente est inférieur à 0,5 W/kg, le procédé comprenant les étapes consistant à :

- disposer la substance ou les substances ($S_1$) dans un premier récipient thermiquement isolant (110),
- mesurer un profil temporel de température ($T_{s1}(t)$) de la substance ou des substances ($S_1$) disposées dans le premier récipient thermiquement isolant (110) pendant une première période de temps initiale (411), en fonction du temps (t),
- déterminer, pendant la première période de temps initiale (411), et en fonction du temps (t), un profil temporel de température (To(t)) de l'environnement (200) dans lequel le premier récipient thermiquement isolant (110) est disposé pendant la première période de temps initiale (411),
- mesurer un profil temporel de température ($T_{s1}(t)$) de la substance ou des substances ($S_1$) disposées dans le premier récipient thermiquement isolant (110) pendant une première période de temps secondaire (421) en fonction du temps (t),
- déterminer, pendant la première période de temps secondaire (421), et en fonction du temps (t), un profil temporel de température (To(t)) de l'environnement (200) dans lequel le premier récipient thermiquement isolant (110) est disposé pendant la première période de temps secondaire (421), et
- déterminer la chaleur ($Q_1$) ou le taux de dégagement de chaleur (q1) de la réaction de la substance ou des substances ($S_1$) disposées dans le premier récipient thermiquement isolant, en utilisant

• les profils temporels de température ($T_{s1}(t)$) de la substance ou des substances ($S_1$) disposées dans le premier récipient thermiquement isolant (110) pendant la première période de temps initiale (411) et la première période de temps secondaire (421),
• le profil temporel de température (To(t)) pendant la première période de temps initiale (411) de l'environnement (200) dans lequel le premier récipient thermiquement isolant (110) est disposé pendant la première période de temps initiale (411), et le profil temporel de température (To(t)) pendant la première période de temps secondaire (421) de l'environnement (205) dans lequel le premier récipient thermiquement isolant (110) est disposé pendant la première période de temps secondaire (421), et
• des informations indiquant la capacité thermique totale $\rho_1 V_1 c_1$ de la substance ou des substances ($S_1$) disposées dans le premier récipient thermiquement isolant (110), dans laquelle $\rho_1$ est la densité de la substance ou des substances, $V_1$ est le volume de la substance ou des substances et $c_1$ est la capacité thermique de la substance ou des substances, dans lequel :

- pendant au moins une partie de la première période de temps initiale (411), la température ($T_{s1}(t)$) de la substance ou des substances ($S_1$) disposées dans le premier récipient thermiquement isolant (110) est différente de la température ($T_O(t)$) de l'environnement (200) dans lequel le premier récipient thermiquement isolant (110) est disposé à ce moment, et
- la température moyenne de l'environnement (200) dans lequel le premier récipient thermiquement isolant (110) est disposé pendant la première période de temps initiale (411) est différente de la température moyenne de l'environnement (205) dans lequel le premier récipient thermiquement isolant (110) est disposé pendant la première période de temps secondaire (421).

2. Procédé selon la revendication 1, dans lequel :

- la première période de temps secondaire (421) débute à un moment déterminé par les conditions suivantes :

• la différence de température absolue entre la substance ($S_1$) et l'environnement (200), $|T_{s1}(t) - (T_O(t)|$, a diminué pour atteindre au maximum 20 % de la différence de température absolue maximale entre la substance ($S_1$) et l'environnement (200), max($|T_{s1}(t) - T_O(t)|$) et/ou
• la première période de temps initiale (411) a duré au moins cinq fois la durée (t$\Delta$T1,max) nécessaire pour atteindre la différence de température absolue maximale, telle qu'elle est mesurée à partir du début de la première période de temps initiale (411), et

- le milieu entourant le premier récipient thermiquement isolant (110) est dans le même état pendant la première période de temps initiale (411) et la première période de temps secondaire (421).

3. Procédé selon la revendication 1 ou 2, comprenant les étapes consistant à :

- disposer la substance ou les substances ou une autre substance ou d'autres substances ($S_2$) dans au moins un second récipient thermiquement isolant (120),

- disposer, pendant une seconde période de temps initiale (412), le second récipient thermiquement isolant (120) dans le même environnement (200) dans lequel le premier récipient thermiquement isolant (110) est disposé pendant au moins une partie de cette période de temps (412),
- mesurer un profil temporel de température ($T_{s2}(t)$) de la substance ou des substances ou de l'autre substance ou des autres substances ($S_2$) disposées dans le second récipient thermiquement isolant (120) pendant la seconde période de temps initiale (412) et en fonction du temps (t),
- déterminer, pendant la seconde période initiale (412), et en fonction du temps (t), un profil temporel de température ($T_O(t)$) de l'environnement (200) dans lequel le second récipient thermiquement isolant (120) est disposé pendant la seconde période de temps initiale (412),
- disposer, pendant une seconde période de temps secondaire (422), le second récipient thermiquement isolant (120) dans le même environnement (205) dans lequel le premier récipient thermiquement isolant (110) est disposé pendant au moins une partie de cette période de temps (422),
- mesurer un profil temporel de température ($T_{s2}(t)$) de la substance ou des substances ou de l'autre substance ou des autres substances ($S_2$) disposées dans le second récipient thermiquement isolant (120) pendant la seconde période de temps secondaire (422) et en fonction du temps (t),
- déterminer, pendant la seconde période de temps secondaire (422), et en fonction du temps (t), un profil temporel de température ($T_O(t)$) de l'environnement (205) dans lequel le second récipient thermiquement isolant (120) est disposé pendant la seconde période de temps secondaire (422), et
- déterminer la chaleur ou le taux de dégagement de chaleur de la réaction de la substance ou des substances ou de l'autre substance ou des autres substances ($S_2$) disposées dans le second récipient thermiquement isolant (120), en utilisant :

   • les profils temporels de température ($T_{s2}(t)$) de la substance ou des substances ou de l'autre substance ou des autres substances ($S_2$) disposées dans le second récipient thermiquement isolant (120) pendant la seconde période de temps initiale (412) et la seconde période de temps secondaire (422),
   • le profil temporel de température (To(t)) pendant la seconde période de temps initiale (412) de l'environnement (200) dans lequel le second récipient thermiquement isolant (110) est disposé pendant la seconde période de temps initiale (412), et le profil temporel de température ($T_O(t)$) pendant la seconde période de temps secondaire (422) de l'environnement (205) dans lequel le second récipient thermiquement isolant (120) est disposé pendant la seconde période de temps secondaire (422), et
   • des informations indiquant la capacité thermique totale de la substance ou des substances ou de l'autre substance ou des autres substances ($S_2$) disposées dans le second récipient thermiquement isolant (120).

4. Procédé selon la revendication 3, dans lequel

   - la première période de temps initiale (411) chevauche au moins en partie la seconde période de temps initiale (412), par exemple sur au moins 10 % de la durée de la plus courte des périodes, et
   - pendant la période de temps dans laquelle la première période de temps initiale (411) et la seconde période de temps initiale (412) se chevauchent, le premier récipient thermiquement isolant (110) et le second récipient thermiquement isolant (120) sont disposés dans le même environnement (200),
   de préférence dans lequel
   - la première période de temps initiale (411) est la même que la seconde période de temps initiale (412).

5. Procédé selon la revendication 3 ou 4, dans lequel

   - la première période de temps secondaire (421) chevauche au moins en partie la seconde période de temps secondaire (422), par exemple sur au moins 10 % de la durée de la plus courte des périodes, et
   - pendant la période de temps dans laquelle la première période de temps secondaire (421) et la seconde période de temps secondaire (422) se chevauchent, le premier récipient thermiquement isolant (110) et le second récipient thermiquement isolant (120) sont disposés dans le même environnement (205),
   de préférence dans lequel
   - la première période de temps secondaire (421) est la même que la seconde période de temps secondaire (422).

6. Procédé selon l'une quelconque des revendications 1 à 5, dans lequel :

   - les réactions de la substance ou des substances ($S_1$) dans le premier récipient thermiquement isolant (110) sont si lentes qu'à tout moment pendant la première période de temps initiale (421), la valeur absolue spécifique du taux de dégagement de chaleur $|q_1(t)/(\rho_1 V_1)|$ est inférieure à 2 W/kg, et/ou la valeur absolue de la cadence de variation de température adiabatique $|q_1(t)/(\rho_1 V_1 c_1)|$ est inférieure à 2 °C/h et/ou
- la première période de temps initiale (411) dure au moins 50 heures.

7. Procédé selon l'une quelconque des revendications 1 à 6, dans lequel

- la valeur moyenne dans le temps, pendant la première période de temps secondaire (421), du profil temporel de température $(T_O(t))$ de l'environnement (205), dans lequel le premier récipient thermiquement isolant (110) est disposé durant la première période de temps secondaire (421) est inférieure à la valeur moyenne dans le temps, pendant la première période de temps initiale (411), du profil temporel de température $(T_O(t))$ de l'environnement (200) dans lequel le premier récipient thermiquement isolant (110) est disposé durant la première période de temps initiale (411), facultativement d'au moins 1 °C, et facultativement au maximum de 50 °C.

8. Procédé selon l'une quelconque des revendications 1 à 7, dans lequel, pendant au moins la moitié de la première période de temps initiale (411) :

- la température de l'environnement $(T_O(t))$ dans lequel le premier récipient thermiquement isolant (110) est disposé durant la première période de temps initiale (411) est de 20 °C à 100 °C, et/ou
- la température $(T_{s1}(t))$ de la substance ou des substances $(S_1)$ disposées dans le premier récipient thermiquement isolant (110) durant la première période de temps initiale (411) est de 20 °C à 150 °C.

9. Procédé selon l'une quelconque des revendications 1 à 8, dans lequel

- le premier récipient thermiquement isolant (110) est disposé dans un même environnement régulé en température (200, 205) d'un dispositif (210) entourant l'environnement régulé en température pendant :

- la première période de temps initiale (411), et
- la première période de temps secondaire (421).

10. Procédé selon l'une quelconque des revendications 1 à 9, dans lequel

- la substance ou des substances $(S_1)$ comprennent de la bio-huile, telle que de la bio-huile pouvant être fabriquée à partir de matières premières d'origine biologique dans un procédé industriel,
de préférence
- la substance ou les substances $(S_1)$ comprennent de l'huile pyrolytique.

11. Procédé selon l'une quelconque des revendications 1 à 10, dans lequel, à tout moment pendant la première période de temps initiale (411),

- la température $(T_O(t))$ du premier environnement et la température $(T_{s1}(t))$ de la substance ou des substances $(S_1)$ sont inférieures à 250 °C, et
- la différence de température absolue entre la substance $(S_1)$ et le premier environnement (200), c'est-à-dire $|T_{s1}(t) - T_O(t)|$ est inférieure à 150 °C, et dans lequel
- le transfert de chaleur, y compris le transfert de chaleur radiatif entre la substance $(S_1)$ dans le premier récipient thermiquement isolant (110) et l'environnement (200) peut être considéré comme proportionnel à la différence de température $(T_{s1}(t) - T_O(t))$ entre le premier récipient thermiquement isolant (110) et l'environnement (200).

12. Procédé selon l'une quelconque des revendications 1 à 11, dans lequel

- au moins 90 % en poids de la substance ou des substances $(S_1)$ est sous forme liquide, et
- le premier récipient thermiquement isolant (110) est sélectionné de manière à ce que la capacité thermique du premier récipient thermiquement isolant (110) soit au maximum de 15 % de la capacité thermique totale $(\rho_1 V_1 c_1)$ de la substance ou des substances $(S_1)$ disposées dans le premier récipient thermiquement isolant (110).

13. Procédé selon l'une quelconque des revendications 1 à 12, comprenant les étapes consistant à :

- déterminer au moins deux paramètres cinétiques de réaction en se servant de la chaleur $(Q_1)$ ou du taux de dégagement de chaleur $(q_1)$ produit par la réaction lente,

- estimer, en se servant des paramètres cinétiques de réaction, au moins un des suivants :

- une durée de stockage telle que la chaleur de réaction résiduelle de la réaction lente soit inférieure à un niveau critique, et
- un taux de transfert de chaleur critique tel que la valeur absolue du taux de dégagement de chaleur de la réaction lente soit inférieure au taux de transfert de chaleur critique pendant au moins 75 % d'une durée de stockage,

- stocker une matière similaire à la substance ou aux substances ($S_1$)

- pendant au moins la durée de stockage et/ou
- dans un récipient équipé d'un équipement de transfert de chaleur ayant la possibilité de compenser un taux de dégagement de chaleur dont la valeur absolue est au maximum le taux de transfert de chaleur critique, et

- ensuite, débloquer, expédier ou transmettre la matière similaire à la substance ou aux substances ($S_1$) à un client.

14. Programme informatique qui, lorsqu'il est exécuté sur un ordinateur, engendre la mise en œuvre des opérations suivantes par l'ordinateur, consistant à :

- recevoir des informations indiquant un profil temporel de température ($T_{s1}(t)$) d'une substance ou de substances ($S_1$) disposées dans un premier récipient thermiquement isolant (110) pendant une première période de temps initiale (411) et en fonction du temps (t),
- recevoir des informations indiquant un profil temporel de température ($T_O(t)$), pendant la première période de temps initiale (411) et en fonction du temps (t), de l'environnement (200) dans lequel le premier récipient thermiquement isolant (110) est disposé pendant la première période de temps initiale (411),
- recevoir des informations indiquant un profil temporel de température ($T_{s1}(t)$) de la substance ou des substances ($S_1$) disposées dans le premier récipient thermiquement isolant (110) pendant une première période de temps secondaire (421) et en fonction du temps (t),
- recevoir des informations indiquant un profil temporel de température ($T_O(t)$), pendant la première période de temps secondaire (421) et en fonction du temps (t), de l'environnement (205) dans lequel le premier récipient thermiquement isolant (110) est disposé pendant la première période de temps secondaire (421), et
- déterminer la chaleur ($Q_1$) ou le taux de dégagement de chaleur ($q_1$) de la réaction de la substance ou des substances ($S_1$) disposées dans le premier récipient thermiquement isolant (110), en utilisant

• les profils temporels de température ($T_{s1}(t)$) de la substance ou des substances ($S_1$) disposées dans le premier récipient thermiquement isolant (110) pendant la première période de temps initiale (411) et pendant la première période de temps secondaire (421),
• le profil temporel de température ($T_O(t)$), pendant la première période de temps initiale (411), de l'environnement (200) dans lequel le premier récipient thermiquement isolant (110) est disposé pendant la première période de temps initiale (411), et le profil temporel de température ($T_O(t)$), pendant la première période de temps secondaire (421), de l'environnement (205) dans lequel le premier récipient thermiquement isolant (110) est disposé pendant la première période de temps secondaire (421), et
• des informations indiquant la capacité thermique totale $\rho_1 V_1 c_1$ de la substance ou des substances ($S_1$) agencées dans le premier récipient thermiquement isolant (110), dans laquelle $\rho_1$ est la densité de la substance ou des substances, $V_1$ est le volume de la substance ou des substances et $c_1$ est la capacité thermique de la substance ou des substances, et dans lequel :

- pendant au moins une partie de la première période de temps initiale (411), la température ($T_{s1}(t)$) de la substance ou des substances ($S_1$) disposées dans le premier récipient thermiquement isolant (110) est différente de la température ($T_O(t)$) de l'environnement (200) dans lequel le premier récipient thermiquement isolant (110) est disposé à ce moment, et
- la température moyenne de l'environnement (200) dans lequel le premier récipient thermiquement isolant (110) est disposé pendant la première période de temps initiale (411) est différente de la température moyenne de l'environnement (205) dans lequel le premier récipient thermiquement isolant (110) est disposé pendant la première période de temps secondaire (421).

**15.** Programme informatique selon la revendication 14, qui, lorsqu'il est exécuté sur un ordinateur, engendre en outre la mise en œuvre des opérations suivantes par l'ordinateur, consistant à :

- recevoir des informations indiquant un profil temporel de température ($T_{s2}(t)$) de la substance ou des substances ou d'une autre substance ou d'autres substances ($S_2$) disposées dans un second récipient thermiquement isolant (120) pendant une seconde période de temps initiale (412) et en fonction du temps (t),
- recevoir des informations indiquant un profil temporel de température ($T_O(t)$), pendant la seconde période de temps initiale (412) et en fonction du temps (t), de l'environnement (200) dans lequel le second récipient thermiquement isolant (120) est disposé pendant la seconde période de temps initiale (412),
- recevoir des informations indiquant un profil temporel de température ($T_{s2}(t)$) de la substance ou des substances ou de l'autre substance ou des autres substances ($S_2$) disposées dans le second récipient thermiquement isolant (120) pendant une seconde période de temps secondaire (422) et en fonction du temps (t),
- recevoir des informations indiquant un profil temporel de température ($T_O(t)$), pendant la seconde période de temps secondaire (422) et en fonction du temps (t), de l'environnement (205) dans lequel le second récipient thermiquement isolant (120) est disposé pendant la seconde période de temps secondaire (422), et
- déterminer la chaleur ($Q_2$) ou le taux de dégagement de chaleur ($q_2$) de la réaction de la substance ou des substances ou de l'autre substance ou des autres substances ($S_2$) disposées dans le second récipient thermiquement isolant (120), en utilisant

• les profils temporels de température ($T_{s2}(t)$) de la substance ou des substances ou de l'autre substance ou des autres substances ($S_2$) disposées dans le second récipient thermiquement isolant (120) pendant la seconde période de temps initiale (412) et pendant la seconde période de temps secondaire (422),
• le profil temporel de température ($T_O(t)$), pendant la seconde période de temps initiale (412), de l'environnement (200) dans lequel le second récipient thermiquement isolant (120) est disposé pendant la seconde période de temps initiale (412), et le profil temporel de température ($T_O(t)$) pendant la seconde période de temps secondaire (422) de l'environnement (205) dans lequel le second récipient thermiquement isolant (120) est disposé pendant la seconde période de temps secondaire (422), et
• des informations indiquant la capacité thermique totale de la substance ou des substances ou de l'autre substance ou des autres substances ($S_2$) disposées dans le second récipient thermiquement isolant (120).

Fig. 1

Fig. 2

Fig. 3

Fig. 4

Fig. 5a

411

412

421

422

Time

Fig. 5b

411

412

421

422

Time

Fig. 5c

411

412

421

422

Time

Fig. 5d

411

412

421

422

Time

**EP 3 040 715 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 4414082 A1
- US 4783174 A